# EUROPEAN PATENT SPECIFICATION

(11) **EP 3 413 891 B1**
(45) Date of publication and mention of the grant of the patent: **03.01.2024**
(21) Application number: 17749950.6
(22) Date of filing: 09.02.2017
(51) Int. Cl.: C07D 405/12, C07D 307/79, C07C 55/10, A61K 31/4525, A61P 1/00

(54) **PROCESSES FOR THE PREPARATION OF HIGHLY PURE PRUCALOPRIDE SUCCINATE**
VERFAHREN ZUR HERSTELLUNG VON HOCHREINEM PRUCALOPRIDSUCCINAT
PROCÉDÉS POUR LA PRÉPARATION DE SUCCINATE DE PRUCALOPRIDE TRÈS PUR

(30) Priority: 11.02.2016 IN 201641004815
(43) Date of publication of application: 19.12.2018
(73) Proprietor: Symed Labs Limited, Banjara Hills, Hyderabad Telangana 500034 (IN)
(72) Inventor: MOHAN RAO, Dodda, Hyderabad Telangana 500033 (IN); VENUGOPAL, Bingi, Karimnagar Telangana 505468 (IN)
(74) Representative: Lederer & Keller Patentanwälte Partnerschaft mbB
(86) International application number: PCT/IB2017/050696
(87) International publication number: WO 2017/137910

(56) References cited:
- EP-A1- 0 389 037
- WO-A1-96/16060
- WO-A2-2011/128784
- CN-A- 102 295 594
- CN-A- 103 570 699
- CN-A- 103 664 912
- CN-A- 104 016 949
- CN-A- 104 193 730
- CN-A- 105 294 620
- IN-A- 1 203 516
- US-A- 5 185 335
- US-B1- 6 294 555
- GONG, Y. ET AL.: "Synthesis of prucalopride succinate", CHINESE JOURNAL OF PHARMACEUTICALS, vol. 46, no. 11, 2015, pages 1158-1160, XP009515287, ISSN: 1001-8255, DOI: 10.16522/J.CNKI.CJPH.2015.11.002
- KAKIGAMI, T. ET AL.: "Synthesis and Structure-Activity Relationship of 3-Substituted Benzamide, Benzo[b]furan-7-carboxamide, 2,3-Dihydrobenzo[b]furan-7-carboxamide, and Indole-5-carboxamide Derivatives as Selective Serotonin 5-HT4 Receptor Agonists", CHEMICAL AND PHARMACEUTICAL BULLETIN, vol. 46, no. 1, 1998, pages 42-52, XP055612772, ISSN: 0009-2363, DOI: 10.1248/cpb.46.42
- Gabrio Bassotti ET AL: "Prucalopride succinate for the treatment of constipation: an update", Expert Review of Gastroenterology & Hepatology, vol. 10, no. 3, 29 January 2016 (2016-01-29), pages 291-300, XP055612833, UK ISSN: 1747-4124, DOI: 10.1586/17474124.2016.1129897

## Description

### FIELD OF THE INVENTION

The present invention relates to purification processes for producing highly pure prucalopride succinate salt.

### BACKGROUND OF THE INVENTION

U.S. Patent No. 5,854,260 discloses a variety of benzofuran-carboxamide derivatives, specifically prucalopride chemically named as 4-amino-5-chloro-2,3-dihydro-N-[1-(3-methoxypropyl)-4-piperidinyl]-7-benzofurancarboxamide, and pharmaceutically acceptable salts and hydrates thereof, processes for the preparation, pharmaceutical compositions, and method of use thereof. Prucalopride is a gastroprokinetic agent acting as a selective serotonin 5-HT₄ receptor agonist which targets the impaired motility associated with chronic constipation, thus normalizing bowel movements. Prucalopride and its pharmaceutically acceptable salts and hydrates thereof show superior enterokinetic properties and are used in the treatment of conditions involving an impaired motility of the intestine, especially of the colon. Prucalopride is represented by the following structural formula 1:

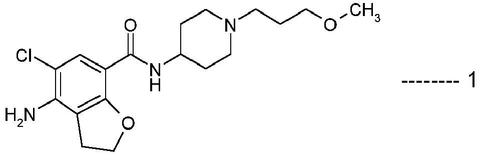

Prucalopride was approved by the EMEA for use in Europe for the treatment of chronic constipation and it is sold under the trade name RESOLOR^{™}. It is orally administered as tablets containing 1 mg and 2 mg of prucalopride (as prucalopride succinate salt).

Various processes for the preparation of benzofurancarboxamide derivatives having gastrointestinal motility stimulating properties, preferably prucalopride, and their intermediates, and pharmaceutically acceptable salts and hydrates thereof, are disclosed in U.S. Patent Nos. 5,854,260; 5,374,637; 5,262,418; 5,185,335; 5,459,161; 8,063,069; and Chemical and Pharmaceutical Bulletin 46(1), 42-52, 1998; and Journal of Heterocyclic Chemistry 17, 1333-1335 (1980).

U.S. Patent No. 5,854,260 (hereinafter referred to as the US'260 patent) describes several synthetic routes for preparing prucalopride. According to one synthetic process, prucalopride was prepared by the condensation of 4-amino-5-chloro-2,3-dihydro-7-benzofurancarboxylic acid with 1-(3-methoxypropyl)-4-piperidinamine in the presence of ethyl chloroformate and N,N-diethylethanamine in trichloromethane to produce a reaction mass, followed by usual work-up and subsequent treatment with isopropanolic-HCl solution to produce prucalopride monohydrochloride salt.

According to another synthetic process as described in the US'260 patent (Example 2), prucalopride is prepared by reacting 4-amino-5-chloro-2,3-dihydro-N-(4-piperidinyl)-7-benzofurancarboxamide with 1-chloro-3-methoxypropane (or its sulfonyloxy analog) in a reaction inert solvent such as a dipolar aprotic solvent, e.g. N,N-dimethylformamide in the presence of potassium iodide and N,N-diethylethanamine at a temperature of about 50°C overnight to produce a reaction mass, followed by usual work-up and subsequent column chromatographic purification over silica gel to produce prucalopride, which is then converted into its hydrochloride salt by treating with isopropanolic-HCl solution.

However, the above process for the preparation of Prucalopride hydrochloride suffers from several drawbacks since the yield of the product obtained is very low (Reported Yield: 35%) and the process requires expensive and slow column chromatographic purifications and thereby making the process commercially not feasible.

According to another synthetic process as described in the US'260 patent, prucalopride is prepared by the condensation of 4-amino-5-chloro-2,3-dihydro-7-benzofurancarboxylic acid with 1-(3-methoxypropyl)-4-piperidinamine under nitrogen atmosphere in the presence of 1,1'-carbonylbis-1H-imidazole in tetrahydrofuran solvent to produce a reaction mass, followed by tedious work-up procedures to produce prucalopride free base as a residue, which is then stirred with water and then dried to yield prucalopride monohydrate (melting point: 90.7°C).

According to US'260 patent, prucalopride succinate is prepared by dissolving prucalopride monohydrate in warm ethanol, followed by the addition of a solution of succinic acid in aqueous ethanol. The resulting mixture was stirred for 24 hours at 23°C and the precipitated solid is filtered, washed and then dried the solid under vacuum for 72 hours at 55°C to yield prucalopride succinate (melting point: 197.2°C).

However, the processes for preparation of prucalopride as described in the aforementioned prior art suffer from several disadvantages such as the use of column chromatographic purifications, and tedious work-up procedures using excess amounts of solvents which generate a large quantity of chemical waste which is difficult to treat. The main drawback of the processes described in the US'260 patent is that the prucalopride succinate obtained by the process does not have satisfactory purity and the yields of the product obtained are very low.

Chemical and Pharmaceutical Bulletin 46(1), 42-52 (hereinafter referred to as the 'CPB Journal') discloses a process for the preparation of 4-amino-5-chloro-2,3-dihydro-7-benzofurancarboxylic acid. The synthesis is depicted in scheme 1:

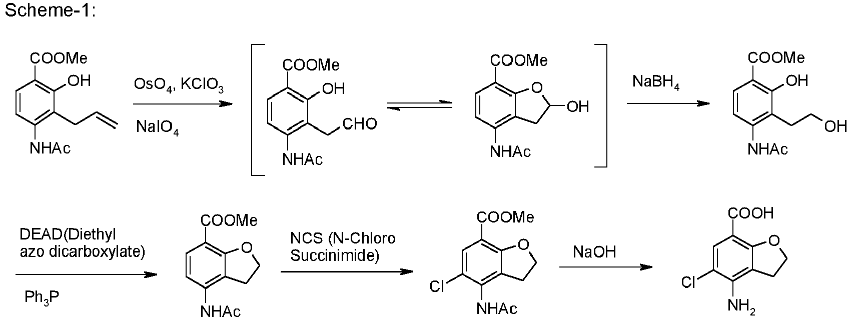

As per the process reported in the CPB Journal, the prucalopride intermediate, 4-amino-5-chloro-2,3-dihydro-7-benzofurancarboxylic acid, is prepared by oxidizing methyl 4-acetylamino-3-allyl-2-hydroxybenzoate with osmium tetroxide in diethyl ether and water, followed by stirring the mixture for 10 minutes at 20-25°C and subsequent addition of sodium periodate. The resulting mixture is stirred for 12 hours at 20-25°C and the resulting precipitate is collected by filtration to produce a mixture of methyl 4-acetylamino-3-formylmethyl-2-hydroxybenzoate and methyl 4-acetylamino-2,3-dihydro-2-hydroxybenzofuran-7-carboxylate (melting point: 179-180°C). The resulting mixture is reduced with sodium borohydride to produce methyl 4-acetylamino-2-hydroxy-3-(2-hydroxyethyl)benzoate. The resulting dihydroxy compound is then reacted with diethyl azodicarboxylate (DEAD) in the presence of triphenylphosphine in tetrahydrofuran, and the mixture is stirred for 1 hour at 20-25°C. The solvent is removed from the mass in vacuo, and the residue is purified by silica gel column chromatography (ethyl acetate : hexane = 1:1) to produce methyl 4-acetylamino-2,3-dihydrobenzo[b]furan-7-carboxylate. This compound is reacted with N-chlorosuccinimide to produce methyl 4-acetylamino-5-chloro-2,3-dihydro-benzo[b]furan-7-carboxylate, followed by alkaline hydrolysis with sodium hydroxide in methanol to yield 4-amino-5-chloro-2,3-dihydro-7-benzofurancarboxylic acid.

The process for the preparation of prucalopride intermediate, 4-amino-5-chloro-2,3-dihydro-7-benzofurancarboxylic acid intermediate, described in the CPB Journal suffers from several disadvantages such as the use of highly expensive and hazardous reagents; formation of excess amounts of diethyl hydrazodicarboxylate as by-product; use of tedious and cumbersome procedures like prolonged reaction time periods, column chromatographic purifications, multiple isolations/ re-crystallizations, and thus resulting in a poor product yield and quality.

The major drawback of the process described in the CPB Journal is that the by-product diethyl hydrazodicarboxylate, formed during the reaction of methyl 4-acetylamino-2-hydroxy-3-(2-hydroxyethyl)benzoate with diethyl azodicarboxylate (DEAD), needs to be removed by column chromatographic purifications, and thus resulting in a poor product yield. The present inventors have found that the alkaline hydrolysis reaction of methyl 4-acetylamino-5-chloro-2,3-dihydro-benzo[b]furan-7-carboxylate with sodium hydroxide in methanol described in the CPB Journal does not yield the desired intermediate compound 4-amino-5-chloro-2,3-dihydro-7-benzofurancarboxylic acid. Whereas, the alkaline hydrolysis reaction described in the CPB Journal yields a partially hydrolyzed 4-acetylamino-5-chloro-2,3-dihydro-7-benzofurancarboxylic acid as the amide group remains intact.

The methods involving column chromatographic purifications are generally undesirable for large-scale operations, thereby making the process commercially unfeasible.

U.S. Patent No. 5,185,335 (hereinafter referred to as '335 patent) describes a process for the preparation of 4-amino-5-chloro-2,3-dihydro-N-(4-piperidinyl)-7-benzofurancarboxamide by reacting ethyl 4-[[(4-amino-5-chloro-2,3-dihydro-N-(4-piperidinyl)-7-benzofurancarboxamide with potassium hydroxide in 2-propanol solvent. The reported yield for this reaction step is only 83.5%.

The Chinese patent application No. CN 104193730 A (hereinafter referred to as CN'730 patent application) describes process for the crystallization of Prucalopride succinate using methanol, ethanol or N,N-dimethylformamide or any one of them with water as a mixed solvent. Example 2 of CN'730 describes process for purification of Prucalopride succinate using methanol/water as solvent.

The main drawback of the processes for the preparation of Prucalopride succinate as described in the above mentioned literature is that the Prucalopride succinate obtained by the processes described therein does not have satisfactory purity (e.g., about 98.5% purity by HPLC) and found to be contaminated with unacceptable amounts of impurities and other solid state forms and solvated forms.

Based on the aforementioned drawbacks, the prior art processes have been found to be unsuitable for the preparation of Prucalopride succinate and its intermediates at commercial scale operations. Moreover, the Prucalopride succinate obtained according to the prior art processes is an impure form and therefore not suitable for pharmaceutical formulations and therapeutic use thereof.

A need remains for an improved, commercially viable and environmentally friendly process of preparing Prucalopride succinate and its intermediates with high yields and purity, to resolve the problems associated with the processes described in the prior art, and that will be suitable for large-scale preparation.

### SUMMARY OF THE INVENTION

The present inventors have surprisingly and unexpectedly found that highly pure Prucalopride succinate essentially free of other polymorphic forms and solvated/hydrated forms, can be obtained by providing a solution of Prucalopride succinate in a solvent medium comprising water and isopropyl alcohol at reflux, subjecting the solution to carbon treatment, cooling the solution to below about room temperature to cause crystallization, and then collecting the highly pure crystalline form of Prucalopride succinate.

The processes disclosed herein advantageously produce the crystalline form of Prucalopride succinate with a purity of greater than about 99.5%, specifically greater than about 99.7%, and most specifically greater than about 99.9% as measured by HPLC.

It has been further surprisingly and unexpectedly found that Prucalopride or an acid addition salt thereof can be prepared in high purity and with high overall yield (about 80% yield) by reacting 4-amino-5-chloro-2,3-dihydro-N-(4-piperidinyl)-7-benzofurancarboxamide with 1-chloro-3-methoxypropane in a reaction inert solvent in the presence of a base and a suitable catalyst such as potassium iodide when the reaction is carried out at a temperate of above 70°C, preferably at 80°C to 100°C. Moreover, it has been unexpectedly found that the reaction is advantageously completed within 4-6 hours when the reaction is carried out at a temperature of 90°C to 95°C.

It has been further surprisingly and unexpectedly found that 4-amino-5-chloro-2,3-dihydro-N-(4-piperidinyl)-7-benzofurancarboxamide can be prepared in high purity and with high yield (about 96.8% yield) by deprotecting ethyl 4-[[(4-amino-5-chloro-2,3-dihydro-7-benzofuranyl)carbonyl]amino]-1-piperidinecarboxylate with an acid, preferably sulfuric acid. Whereas the yield of the product obtained by deprotection of ethyl 4-[[(4-amino-5-chloro-2,3-dihydro-7-benzofuranyl)carbonyl]amino]-1-piperidinecarboxylate with potassium hydroxide as reported in the prior art (Example 3, step-(b) of US 5,185,335) is comparatively very low (only 83.5% yield).

In one aspect, provided herein are simple, commercially viable and consistently reproducible purification processes for the preparation of highly pure Prucalopride succinate.

In another aspect, disclosed herein are improved and industrially advantageous processes for the preparation of Prucalopride and its intermediates, in high yield and with high purity, using novel intermediates. The processes disclosed herein avoid the tedious and cumbersome procedures of the prior processes, thereby resolving the problems associated with the processes described in the prior art, which are more convenient to operate at lab scale and in commercial scale operations. The processes disclosed herein also avoid the use of prolonged reaction time periods, column chromatographic purifications, multiple isolations/ re-crystallizations, and thus resulting in increased product yield and quality.

### BRIEF DESCRIPTION OF THE DRAWINGS

**Figure 1** is a characteristic powder X-ray diffraction (XRPD) pattern of Crystalline Form of highly pure Prucalopride Succinate prepared according to the present invention.
**Figure 2** is a characteristic infra-red (IR) spectrum of Crystalline Form of highly pure Prucalopride Succinate prepared according to the present invention.
**Figure 3** is a characteristic Differential Scanning Calorimetric (DSC) thermogram of Crystalline Form of highly pure Prucalopride Succinate prepared according to the present invention.

### DETAILED DESCRIPTION OF THE INVENTION

According to one aspect, there is provided a process for purification of Prucalopride succinate, comprising:
a) providing a solution of Prucalopride succinate in a solvent medium comprising an organic solvent and water at a temperature of above 40°C, wherein the solvent medium is selected from the group consisting of a solvent medium comprising isopropyl alcohol and water; a solvent medium comprising isopropyl alcohol, methanol and water; and a solvent medium comprising acetone and water;
b) subjecting the solution obtained in step-(a) to carbon treatment to obtain a clear filtrate;
c) cooling the filtrate obtained in step-(b) at a temperature below 35°C to cause crystallization; and
d) recovering the highly pure Prucalopride Succinate obtained in step-(c).

Preferably, the solvent medium used in step-(a) is a solvent medium comprising isopropyl alcohol and water.

The use of the specific solvent medium comprising water and isopropyl alcohol employed herein for purification of Prucalopride succinate surprisingly and unexpectedly produces the product with high purity.

Usually, the amount of solvent medium employed in step-(a) is 5 volumes to 15 volumes, specifically about 10 volumes to about 12 volumes, with respect to the quantity of crude Prucalopride succinate used.

In one embodiment, the amount of isopropyl alcohol employed in step-(a) is 5 to 15 volumes, specifically about 10 to 12 volumes, with respect to the volume of water used.

In another embodiment, the amount of acetone employed in step-(a) is about 5 to 10 volumes, specifically about 6 to 8 volumes, with respect to the volume of water used.

Step-(a) of providing a solution of Prucalopride succinate includes dissolving or extracting an impure Prucalopride succinate in the solvent medium used in step-(a), or obtaining an existing solution from a previous processing step.

Unless otherwise specified, the impure Prucalopride succinate as used herein above as starting material can be obtained, for example, by the process described in the U.S. Patent No. 5,854,260, or by the processes described hereinafter.

In another embodiment, the Prucalopride succinate is dissolved or extracted in the solvent medium used in step-(a) at a temperature of about 45°C to reflux temperature of the solvent medium used, specifically at reflux temperature of the solvent medium used. After complete dissolution of Prucalopride succinate, the resulting solution is stirred at the reflux temperature for at least 5 minutes, and specifically for about 10 minutes to about 30 minutes.

In another embodiment, the solution in step-(a) is also prepared by suspending Prucalopride succinate in the solvent medium used in step-(a) at room temperature, followed by heating the suspension at a temperature of about 45°C to reflux temperature to form a clear solution, specifically at reflux temperature of the solvent medium used. After complete dissolution of Prucalopride succinate, the resulting solution is stirred at reflux temperature for at least 5 minutes, and specifically for about 10 minutes to about 30 minutes.

In another embodiment, the solution in step-(a) is also prepared by suspending Prucalopride succinate in the organic solvent used, followed by heating the suspension at a temperature of 45°C to the reflux temperature of the organic solvent used, specifically at the reflux temperature of the organic solvent used, to form a hot suspension and then adding water to the hot suspension to form a clear solution.

In another embodiment, the solution in step-(a) is also prepared by treating Prucalopride free base or Prucalopride monohydrate with succinic acid in a suitable inert solvent to produce a reaction mass containing Prucalopride succinate, followed by usual work up such as washings, extractions, evaporations or a combination thereof, and then dissolving or extracting the resulting Prucalopride succinate in the solvent medium comprising organic solvent and water used in step-(a).

Alternatively, the solution in step-(a) is also prepared by treating an acid addition salt of Prucalopride with a base to liberate Prucalopride free base, treating the resulting Prucalopride free base with succinic acid in a suitable inert solvent to produce a reaction mass containing Prucalopride succinate, followed by usual work up such as washings, extractions, evaporations or a combination thereof, and then dissolving or extracting the resulting Prucalopride succinate in the solvent medium comprising organic solvent and water used in step-(a).

In another embodiment, the acid addition salt of Prucalopride is derived from a therapeutically acceptable acid such hydrochloric acid, hydrobromic acid, sulfuric acid, nitric acid, oxalic acid, acetic acid, propionic acid, phosphoric acid, succinic acid, maleic acid, fumaric acid, citric acid, glutaric acid, tartaric acid, benzenesulfonic acid, toluenesulfonic acid, malic acid, ascorbic acid, and the like.

The treatment of an acid addition salt with a base is carried out in a solvent and the selection of solvent is not critical. A wide variety of solvents such as chlorinated solvents, alcohols, ketones, hydrocarbon solvents, esters, ether solvents etc., can be used. The base used herein is an organic or inorganic base. Specific organic bases are triethyl amine, trimethylamine, N,N-diisopropylethylamine, N-methylmorpholine and N-methylpiperidine. Specific inorganic bases are ammonia, sodium hydroxide, calcium hydroxide, magnesium hydroxide, potassium hydroxide, lithium hydroxide, sodium carbonate, potassium carbonate, lithium carbonate, sodium tert-butoxide, sodium isopropoxide and potassium tert-butoxide, and more specifically ammonia, sodium hydroxide, potassium hydroxide, sodium carbonate and potassium carbonate.

The carbon treatment in step-(b) is carried out by methods known in the art, for example, by stirring the solution with finely powdered carbon at a temperature of about 40°C to the reflux temperature for at least 5 minutes, specifically at the reflux temperature; and filtering the resulting mixture through charcoal bed to obtain a filtrate containing Prucalopride succinate by removing charcoal. Specifically, finely powdered carbon is a special carbon or an active carbon.

In one embodiment, the crystallization in step-(c) is accomplished by cooling the solution at a temperature of 0°C to 35°C for at least 10 minutes, and more specifically at a temperature of about 20°C to about 30°C for about 15 minutes to about 2 hours.

The recovering in step-(d) is carried out by the methods such as filtration, filtration under vacuum, decantation, centrifugation or a combination thereof.

Surprisingly, the purification processes disclosed herein above advantageously produces the Prucalopride succinate as a crystalline form with high purity.

The highly pure crystalline form of Prucalopride succinate obtained by the processes disclosed herein has a purity of greater than about 99.5%, specifically greater than about 99.8%, and most specifically greater than about 99.9% as measured by HPLC. For example, the purity of the highly pure Prucalopride succinate obtained by the processes disclosed herein is about 99.7% to about 99.99% as measured by HPLC.

According to another aspect, there is disclosed a highly pure crystalline form of Prucalopride succinate having purity greater than about 99.5%, specifically greater than about 99.8%, and most specifically greater than about 99.9% as measured by HPLC.

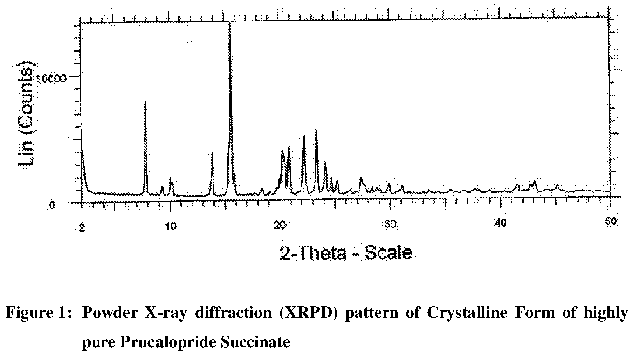

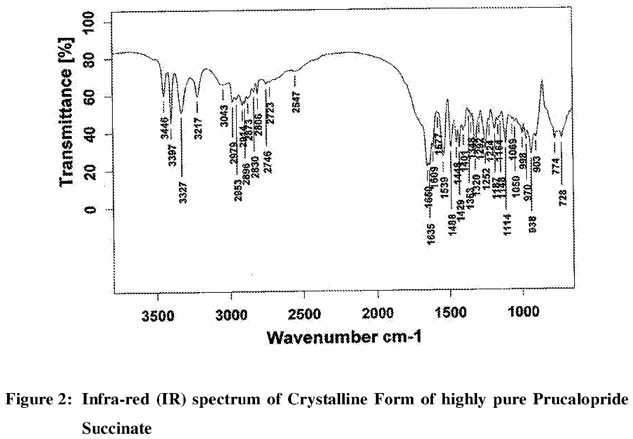

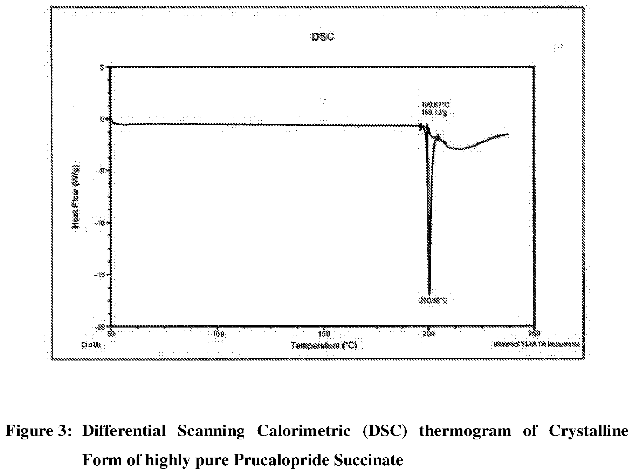

In one embodiment, the highly pure crystalline form of Prucalopride succinate obtained according to the present invention is characterized by an X-ray powder diffraction pattern having peaks expressed as 2-theta angle positions at 7.75, 9.18, 9.97, 10.12, 13.65, 13.77, 15.33, 15.52, 15.80, 18.39, 19.71, 19.96, 20.23, 20.41, 20.85, 22.20, 22.40, 23.36, 24.12, 24.65, 25.17, 27.38, 27.67, 28.37, 28.81, 29.92, 31.13, 41.58, 42.79, 43.20 and 45.24 ± 0.2 degrees substantially in accordance with Figure 1; an infra red (FT-IR) spectrum having main bands at 3446, 3397, 3327, 3217, 2979, 2914, 2806, 1650, 1635, 1609, 1577, 1539, 1488, 1448, 1429, 1401, 1348, 1320, 1252, 1224, 1187, 1164, 1148, 1114, 1069, 1050, 998, 970, 938, 903, 774 and 728 cm⁻¹ ± 2 substantially in accordance with Figure 2; and a Differential Scanning Calorimetric (DSC) thermogram having a sharp endotherm peak at about 200°C substantially in accordance with Figure 3.

In one embodiment, the highly pure crystalline form of Prucalopride succinate obtained by the processes disclosed herein is essentially free from other solid state forms of Prucalopride succinate detectable by the spectral methods typically used, e.g., Powder X-ray diffraction.

The term "highly pure crystalline form of Prucalopride succinate essentially free of other solid state forms" means that no other solid state forms of Prucalopride succinate can be detected within the limits of a powder X-ray diffractometer. The term "other solid state forms of Prucalopride succinate" is intended to mean the solid state forms of Prucalopride succinate other than the crystalline form characterized hereinabove, including crystalline forms, amorphous form, solvated forms, hydrated forms and mixtures thereof.

Unless otherwise specified, the term "crude or impure form of Prucalopride succinate" refers to any form of Prucalopride succinate having purity less than about 99.5%, preferably less than about 99% , and more preferably less than about 98.5% as measured by HPLC.

Unless otherwise specified, the Prucalopride monohydrate used as starting material in the processes described in the present invention can be obtained, for example, by the process described in the U.S. Patent No. 5,854,260, or by the processes described hereinafter.

According to another aspect, the Prucalopride succinate used as starting material in the above purification process of the present invention is prepared by a process comprising:
a) dissolving Prucalopride monohydrate in a solvent medium comprising methanol and isopropyl alcohol at a temperature of 55°C to 65°C to form a clear solution;
b) subjecting the solution obtained in step-(a) to carbon treatment to obtain a clear filtrate at a temperature of 55°C to 65°C to form a clear solution;
c) combining the filtrate obtained in step-(b) with succinic acid; and
d) recovering the Prucalopride Succinate obtained in step-(c).

In one embodiment, the solution in step-(a) is prepared by dissolving Prucalopride monohydrate in the solvent medium comprising methanol and isopropyl alcohol at a temperature of 60°C to 65°C.

The carbon treatment in step-(b) is carried out by methods known in the art, for example, by stirring the solution with finely powdered carbon at a temperature of 55°C to 65°C, preferably at a temperature of about 60°C to about 65°C, for at least 5 minutes, preferably about 10 minutes to 30 minutes; and filtering the resulting mixture through charcoal bed to obtain a filtrate containing Prucalopride by removing charcoal. Specifically, finely powdered carbon is a special carbon or an active carbon.

Combining of the filtrate with succinic acid in step-(c) is done in a suitable order, for example, the filtrate is added to the succinic acid, or alternatively, the succinic acid is added to the filtrate. The addition is, for example, carried out drop wise or in one portion or in more than one portion. The addition is specifically carried out under stirring at a temperature of about 55°C to about 65°C, and most specifically at about 60°C to about 65°C. After completion of the addition process, the resulting mass is stirred at a temperature of below 35°C for at least 10 minutes and specifically at a temperature of about 20°C to about 30°C for about 20 minutes to about 3 hours to cause crystallization.

The recovering in step-(d) is carried out by the methods such as filtration, filtration under vacuum, decantation, centrifugation or a combination thereof.

Disclosed herein is also a novel process for the preparation of Prucalopride of formula 1: 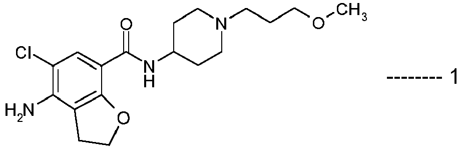 or a pharmaceutically acceptable salt thereof, which comprises:
a) deprotecting the alkyl 4-[[(4-amino-5-chloro-2,3-dihydro-7-benzofuranyl)carbonyl]-amino]-1-piperidinecarboxylate of formula 3: 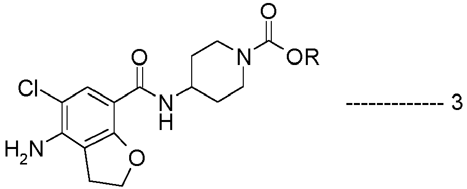
   or a salt thereof, wherein R is an alkyl group having 1 to 5 carbon atoms,
   with an acid in a suitable solvent to produce 4-amino-5-chloro-2,3-dihydro-N-(4-piperidinyl)-7-benzofurancarboxamide of formula 2: 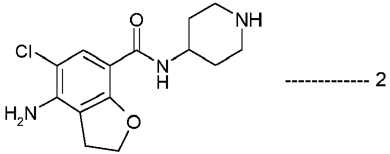 or a salt thereof; and
b) reacting the compound of formula 2 with 1-chloro-3-methoxy-propane in presence of a base, optionally in presence of a suitable catalyst, to produce Prucalopride of formula 1 or a pharmaceutically acceptable salt thereof, and optionally converting the compound of formula I obtained into highly pure Prucalopride succinate.

Exemplary pharmaceutically acceptable salts of the Prucalopride of formula 1 include, but are not limited to, hydrochloride, hydrobromide, sulfate, nitrate, phosphate, acetate, propionate, oxalate, succinate, maleate, fumarate, benzenesulfonate, toluenesulfonate, citrate and tartrate. A most specific pharmaceutically acceptable salt of the Prucalopride of formula 1 is succinate salt.

Unless otherwise specified, the term 'salt' as used herein may include acid addition salts and base addition salts.

Unless otherwise specified, the term 'acid addition salts', as used herein, include the salts that are derived from organic and inorganic acids. For example, the acid addition salts are derived from a therapeutically acceptable acid such as hydrochloric acid, hydrobromic acid, sulfuric acid, nitric acid, oxalic acid, acetic acid, propionic acid, phosphoric acid, succinic acid, maleic acid, fumaric acid, citric acid, glutaric acid, tartaric acid, benzenesulfonic acid, toluenesulfonic acid, malic acid, ascorbic acid, and the like.

Exemplary acid addition salts include, but are not limited to, hydrochloride, hydrobromide, sulphate, nitrate, phosphate, acetate, propionate, oxalate, succinate, maleate, fumarate, benzenesulfonate, toluenesulfonate, citrate, tartrate, and the like.

In one embodiment, the group R in the compound of formula 3 is selected from methyl, ethyl, n-propyl, isopropyl, n-butyl, iso-butyl, tert-butyl, and n-pentyl. Specifically, the group R in the compound of formula 3 is selected from methyl, ethyl, n-propyl and isopropyl; more specifically Ris methyl or ethyl; and most specifically Ris ethyl.

The acid used for deprotection in step-(a) is an organic or an inorganic acid. Exemplary acids include, but are not limited to, hydrochloric acid, hydrobromic acid, sulfuric acid, nitric acid, phosphoric acid, acetic acid, formic acid, trifluoroacetic acid, trifluoroacetic anhydride and the like, or a combination thereof. A most specific acid used for deprotection in step-(a) is sulfuric acid.

For example, the acid used may also be in the form of aqueous solution or in the form of a solution in an organic solvent.

Exemplary solvents used for deprotection in step-(a) include, but are not limited to, water, methanol, ethanol, isopropyl alcohol, tert-butyl alcohol, N,N-dimethylformamide, N,N-dimethylacetamide, dichloromethane, dichloroethane, dioxane, and mixtures thereof. A most specific solvent is water.

In one embodiment, the reaction in step-(a) is carried out at a temperature of about 30°C to the reflux temperature of the solvent used, specifically at a temperature of about 70°C to the reflux temperature of the solvent used, and more specifically at a temperature of about 80°C to the reflux temperature of the solvent used. The reaction time may vary from about 2 hours to about 8 hours.

The reaction mass containing the 4-amino-5-chloro-2,3-dihydro-N-(4-piperidinyl)-7-benzofurancarboxamide of formula 2 or a salt thereof obtained in step-(a) may be subjected to usual work up methods such as a washing, an extraction, a pH adjustment, an evaporation, a layer separation, decolorization, or a combination thereof. The reaction mass may be used directly in the next step to produce the compound of formula 1, or the compound of formula 2 or a salt thereof may be isolated and/or recrystallized and then used in the next step.

In one embodiment, the compound of formula 2 or a salt thereof may be isolated and/or re-crystallized from a suitable solvent by conventional methods such as cooling, seeding, partial removal of the solvent from the solution, by adding an anti-solvent to the solution, evaporation, vacuum distillation, or a combination thereof.

In one embodiment, the reaction in step-(b) is carried out in the presence of a solvent or a mixture of solvents. Exemplary solvents used in step-(b) include, but are not limited to, toluene, xylene, N,N-dimethylformamide, N,N-dimethylacetamide, dichloromethane, dimethylsulfoxide, N-methylpyrrolidone and mixtures thereof. A most specific solvent is N,N-dimethylformamide.

The base used in step-(b) is an organic base. Specifically, the organic bases used in step-(b) include, but are not limited to, methylamine, trimethylamine, tributylamine, triethylamine, diisopropylethylamine, N-methylmorpholine, and 1-alkylimidazole. A most specific organic base is triethylamine.

Preferably, the reaction in step-(b) is carried out in the presence of a suitable catalyst. Specifically, the catalyst used in step-(b) is sodium iodide or potassium iodide.

In one embodiment, the reaction in step-(b) is carried out at a temperature of 60°C to the reflux temperature of the solvent used, specifically at a temperature of 70°C to the reflux temperature of the solvent used, and more specifically at a temperature of 80°C to 100°C. The reaction time may vary from about 2 hours to about 8 hours. The reaction is advantageously completed within 4-6 hours when the reaction is carried out at a temperature of 90°C to 95°C.

The reaction mass containing the Prucalopride of formula 1 or a pharmaceutically acceptable salt thereof obtained in step-(b) may be subjected to usual work up such as a washing, an extraction, an evaporation, a pH adjustment etc., followed by isolation and/or recrystallization from a suitable solvent by conventional methods such as cooling, seeding, partial removal of the solvent from the solution, by adding an anti-solvent to the solution, evaporation, vacuum distillation, or a combination thereof.

The conversion of the Prucalopride of formula 1 or a pharmaceutically acceptable salt thereof into highly pure Prucalopride succinate salt can be carried out as per the processes described herein or by the known methods.

Disclosed herein is also an improved process for the preparation of alkyl 4-[[(4-amino-5-chloro-2,3-dihydro-7-benzofuranyl)carbonyl]-amino]-1-piperidinecarboxylate of formula 3: 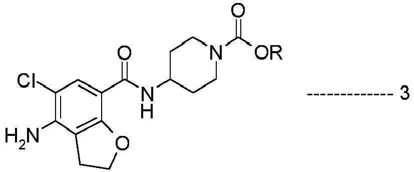 or a salt thereof, wherein R is an alkyl group having 1 to 5 carbon atoms, which comprises:
a) reacting methyl 4-acetylamino-5-chloro-2,3-dihydrobenzo[b]furan-7-carboxylate of formula 7: 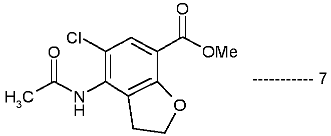 or a salt thereof, with an alkali metal base to produce 4-acetylamino-5-chloro-2,3-dihydrobenzofuran-7-carboxylic acid alkali metal salt of formula 6: 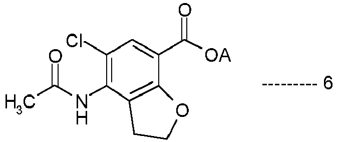 wherein the radical 'A' is an alkali metal;
b) reacting the compound of formula 6 with an acid to produce methyl 4-amino-5-chloro-2,3-dihydrobenzofuran-7-carboxylate of formula 5: 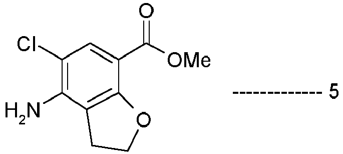 or an acid addition salt thereof;
c) hydrolysis of the compound of formula 5 or an acid addition salt thereof with a suitable inorganic base to produce 4-amino-5-chloro-2,3-dihydro-7-benzofuran-carboxylic acid of formula 4: 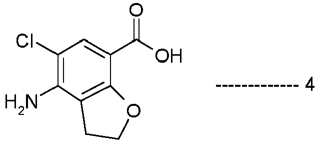 or a salt thereof; and
d) reacting the compound of formula 4 or a salt thereof with an alkyl 4-amino-1-piperidine-carboxylate of formula 10: 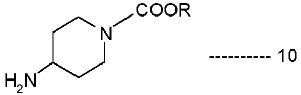 wherein R is as defined above for formula 3, in presence of ethyl chloroformate and an organic base to produce alkyl 4-[[(4-amino-5-chloro-2,3-dihydro-7-benzofuranyl)carbonyl]-amino]-1-piperidinecarboxylate of formula 3 or a salt thereof.

In one embodiment, the group R in the compounds of formulae 3 and 10 is selected from methyl, ethyl, n-propyl, isopropyl, n-butyl, iso-butyl, tert-butyl, and n-pentyl. Specifically, the group R in the compounds of formulae 3 and 10 is selected from methyl, ethyl, n-propyl and isopropyl; more specifically Ris methyl or ethyl; and most specifically R is ethyl.

In one embodiment, the radical 'A' in the compound of formula 6 is sodium or potassium, and most preferably the radical 'A' is sodium.

Exemplary alkali metal bases used in step-(a) include, but are not limited to, hydroxides, bicarbonates and carbonates of alkali metals. Specific alkali metal bases are sodium hydroxide, potassium hydroxide, lithium hydroxide, sodium carbonate, potassium carbonate, sodium bicarbonate, potassium bicarbonate, lithium carbonate; and most specifically, the alkali metal base is sodium hydroxide.

Exemplary solvents used in step-(a) include, but are not limited to, water, methanol, ethanol, isopropyl alcohol, n-butyl alcohol, tert-butyl alcohol, and mixtures thereof. A most specific solvent is a mixture of water and methanol.

In one embodiment, the reaction in step-(a) is carried out at a temperature of about 30°C to the reflux temperature of the solvent used, specifically at a temperature of about 40°C to the reflux temperature of the solvent used, and more specifically at a temperature of about 50°C to about 60°C. The reaction time may vary from about 1 hour to about 5 hours.

The reaction mass containing the 4-acetylamino-5-chloro-2,3-dihydrobenzofuran-7-carboxylic acid alkali metal salt of formula 6 obtained in step-(a) may be subjected to usual work up methods such as a washing, an extraction, a pH adjustment, an evaporation, a layer separation, decolorization, or a combination thereof. The reaction mass may be used directly in the next step to produce the compound of formula 5, or the compound of formula 6 may be isolated and/or recrystallized and then used in the next step.

In one embodiment, the compound of formula 6 is preferably isolated as a solid from a suitable solvent by conventional methods such as cooling, seeding, partial removal of the solvent from the solution, by adding an anti-solvent to the solution, evaporation, vacuum distillation, or a combination thereof.

In one embodiment, the reaction in step-(b) is carried out in the presence of a solvent, preferably methanol.

The acid used for deprotection in step-(b) is an organic or an inorganic acid. Exemplary acids include, but are not limited to, hydrochloric acid, hydrobromic acid, sulfuric acid, nitric acid, phosphoric acid, acetic acid, formic acid, trifluoroacetic acid, trifluoroacetic anhydride and the like, or a combination thereof. A most specific acid used for deprotection in step-(b) is sulfuric acid. For example, the acid used may also be in the form of aqueous solution or in the form of a solution in an organic solvent.

In one embodiment, the reaction in step-(b) is carried out at a temperature of about 25°C to the reflux temperature of the solvent used, specifically at a temperature of about 40°C to the reflux temperature of the solvent used, and more specifically at the reflux temperature of the solvent used. The reaction time may vary from about 1 hour to about 10 hours.

The reaction mass containing the methyl 4-amino-5-chloro-2,3-dihydrobenzofuran-7-carboxylate of formula 5 obtained in step-(b) may be subjected to usual work up methods as described hereinabove. The reaction mass may be used directly in the next step to produce the compound of formula 4, or the compound of formula 5 may be isolated and/or recrystallized and then used in the next step.

In one embodiment, the compound of formula 5 is preferably isolated as a solid from a suitable solvent by conventional methods as described hereinabove.

The reaction in step-(c) is carried out in the presence of a solvent. Exemplary solvents used for reaction in step-(c) include, but are not limited to, water, methanol, ethanol, isopropyl alcohol, n-butyl alcohol, tert-butyl alcohol, and mixtures thereof. A most specific solvent is a mixture of water and methanol.

The inorganic base used for hydrolysis in step-(c) is sodium hydroxide or potassium hydroxide.

In one embodiment, the reaction in step-(c) is carried out at a temperature of about 25°C to the reflux temperature of the solvent used, specifically at a temperature of about 50°C to the reflux temperature of the solvent used, and more specifically at the reflux temperature of the solvent used. The reaction time may vary from about 1 hour to about 4 hours.

The reaction mass containing the 4-amino-5-chloro-2,3-dihydro-7-benzofuran-carboxylic acid of formula 4 obtained in step-(c) may be subjected to usual work up methods as described hereinabove. The reaction mass may be used directly in the next step to produce the compound of formula 3, or the compound of formula 4 may be isolated and/or recrystallized and then used in the next step.

In one embodiment, the compound of formula 4 is isolated as a solid from a suitable solvent by conventional methods as described hereinabove.

In one embodiment, the reaction in step-(d) is carried out in the presence of a solvent or a mixture of solvents. Exemplary solvents used in step-(d) include, but are not limited to, toluene, xylene, dichloromethane, dichloroethane, chloroform and mixtures thereof.

In one embodiment, the organic base used in step-(d) includes, but are not limited to, methylamine, trimethylamine, tributylamine, triethylamine, diisopropylethylamine, N-methylmorpholine, and 1-alkylimidazole. A most specific organic base is triethylamine.

In one embodiment, the reaction in step-(d) is carried out at a temperature of about 0°C to about 50°C and specifically at a temperature of about 0°C to about 30°C. The reaction time may vary from about 2 hours to about 15 hours.

The reaction mass containing the alkyl 4-[[(4-amino-5-chloro-2,3-dihydro-7-benzofuranyl)carbonyl]-amino]-1-piperidinecarboxylate of formula 3 obtained in step-(d) may be subjected to usual work up such as a washing, an extraction, an evaporation, a pH adjustment etc., followed by isolation and/or recrystallization from a suitable solvent by conventional methods as described hereinabove.

Unless otherwise specified, the methyl 4-acetylamino-5-chloro-2,3-dihydrobenzo[b]furan-7-carboxylate of formula 7 used as starting material in the above process can be prepared by the processes described in the present disclosure.

The process for the preparation of methyl 4-acetylamino-5-chloro-2,3-dihydrobenzo[b]furan-7-carboxylate of formula 7 is advantageously carried out without using any expensive and time consuming column chromatographic purifications.

Disclosed herein is also an improved process for the preparation of methyl 4-acetylamino-5-chloro-2,3-dihydrobenzo[b]furan-7-carboxylate of formula 7: 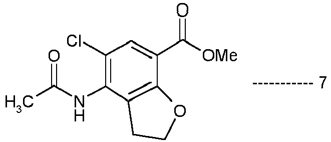 or a salt thereof, which comprises:
a) reacting methyl 4-acetylamino-2-hydroxy-3-(2-hydroxyethyl)benzoate of formula 9: 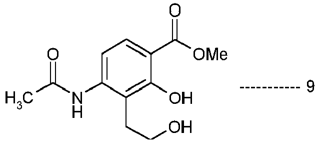 or a salt thereof, with a solution or an organic layer containing diethyl azodicarboxylate or diisopropyl azodicarboxylate and dichloromethane, in presence of triphenylphosphine in a suitable solvent to produce methyl 4-acetylamino-2,3-dihydrobenzo[b]furan-7-carboxylate of formula 8: 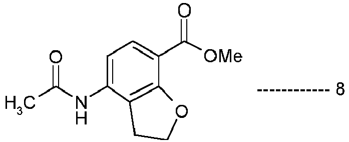 or a salt thereof; and
b) reacting the compound of formula 8 obtained in step-(a) with N-chlorosuccinimide in a suitable solvent to produce the methyl 4-acetylamino-5-chloro-2,3-dihydrobenzo[b]furan-7-carboxylate of formula 7 or a salt thereof.

Exemplary solvents used in step-(a) include, but are not limited to, an ether solvent, a halogenated hydrocarbon solvent, and mixtures thereof.

Specifically, the solvent used in step-(a) is selected from the group consisting of tetrahydrofuran, 2-methyl tetrahydrofuran, dioxane, diethyl ether, diisopropyl ether, methyl tert-butyl ether, monoglyme, diglyme, dichloromethane, dichloroethane, and mixtures thereof. A most specific solvent is a mixture of tetrahydrofuran and dichloromethane.

The reaction in step-(a) is carried out at a temperature of below about 35°C, specifically at a temperature of about 0°C to about 30°C, and most specifically at a temperature of about 20°C to about 30°C. The reaction time may vary from about 1 hour to about 2 hours.

The reaction mass containing the methyl 4-acetylamino-2,3-dihydrobenzo[b]furan-7-carboxylate of formula 8 obtained in step-(a) may be subjected to usual work up methods as described hereinabove.

The reaction mass obtained in step-(a) may be used directly in the next step to produce the compound of formula 7, or the compound of formula 8 may be isolated and/or recovered and then used in the next step.

The compound of formula 8 is recovered by the methods such as filtration, filtration under vacuum, decantation, centrifugation or a combination thereof.

The reaction in step-(b) is carried out in the presence of a solvent, preferably a polar aprotic solvent.

Specifically, the solvent used in step-(b) is selected from the group consisting of N,N-dimethylformamide, N,N-dimethylacetamide, dimethylsulfoxide, N-methylpyrrolidone, and mixtures thereof. A most specific solvent is N,N-dimethylformamide.

The reaction in step-(b) is carried out at a temperature of about 25°C to the reflux temperature of the solvent used, specifically at a temperature of about 40°C to the reflux temperature of the solvent used, and more specifically at a temperature of about 60°C to about 80°C. The reaction time may vary from about 1 hour to about 6 hours.

The reaction mass containing the methyl 4-acetylamino-5-chloro-2,3-dihydrobenzo[b]furan-7-carboxylate of formula 7 obtained in step-(b) may be subjected to usual work up methods as described hereinabove. The reaction mass may be used directly in the process for the preparation of 4-acetylamino-5-chloro-2,3-dihydrobenzofuran-7-carboxylic acid alkali metal salt of formula 6, or the compound of formula 7 may be isolated and/or recovered and then used in the preparation of the compound of formula 6.

The compound of formula 7 is preferably isolated as a solid from a suitable solvent by conventional methods as described hereinabove.

As used herein, the term "reflux temperature" means the temperature at which the solvent or solvent system refluxes or boils at atmospheric pressure.

As used herein, the term "room temperature" or "RT" refer to a temperature of about 20°C to about 35°C, preferably to a temperature of about 25°C to about 30°C.

Disclosed herein is also a 4-acetylamino-5-chloro-2,3-dihydrobenzofuran-7-carboxylic acid alkali metal salt of formula 6: 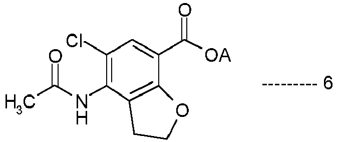 wherein the radical 'A' is an alkali metal.

In one embodiment, the radical 'A' in the compound of formula 6 is sodium or potassium, and most preferably the radical 'A' is sodium.

Unless otherwise specified, the solvent used for isolating and/or recrystallizing the compounds obtained by the processes described in the present application is generally selected from the group consisting of water, an alcohol, a ketone, an ether, an ester, a hydrocarbon, a halogenated hydrocarbon, and mixtures thereof. Specifically, the solvent is selected from the group consisting of water, methanol, ethanol, 1-propanol, isopropyl alcohol, acetone, tetrahydrofuran, 2-methyl-tetrahydrofuran, diisopropyl ether, methyl tert-butyl ether, ethyl acetate, cyclohexane, toluene, xylene, dichloromethane, dichloroethane, chloroform, and mixtures thereof.

Unless otherwise specified, the products and intermediate compounds obtained by the above processes may be further dried in, for example, a Vacuum Tray Dryer, a Rotocon Vacuum Dryer, a Vacuum Paddle Dryer or a pilot plant Rota vapor, to further lower residual solvents. Drying can be carried out under reduced pressure until the residual solvent content reduces to the desired amount such as an amount that is within the limits given by the International Conference on Harmonization of Technical Requirements for Registration of Pharmaceuticals for Human Use ("ICH") guidelines.

In one embodiment, the drying is carried out at atmospheric pressure or reduced pressures, such as below about 26.66 kPa (200 mm Hg), or below about 6.67 kPa (50 mm Hg), at temperatures such as about 35°C to about 90°C, and specifically at about 50°C to about 85°C. The drying can be carried out for any desired time period that achieves the desired result, such as times about 1 to 20 hours. Drying may also be carried out for shorter or longer periods of time depending on the product specifications. Temperatures and pressures will be chosen based on the volatility of the solvent being used and the foregoing should be considered as only a general guidance. Drying can be suitably carried out in a tray dryer, vacuum oven, air oven, or using a fluidized bed dryer, spin flash dryer, flash dryer, and the like.

### INSTRUMENTAL DETAILS:

### X-ray Powder Diffraction (P-XRD):

The X-ray powder diffraction spectrum was measured on a BRUKER AXS D8 FOCUS X-ray powder diffractometer equipped with a Cu-anode (copper-Kα radiation). Approximately 500 mg of sample was gently flattered on a sample holder and scanned from 2 to 50 degrees 2-theta, at 0.03 degrees to theta per step and a step time of 0.4 seconds. The sample was simply placed on the sample holder. The sample was rotated at 30 rpm at a voltage 40 KV and current 35 mA.

### Infra-Red Spectroscopy (FT-IR):

FT-IR spectroscopy was carried out with a Bruker vertex 70 spectrometer. For the production of the KBr compacts approximately 5 mg of sample was powdered with 200 mg of KBr. The spectra were recorded in transmission mode ranging from 3800 cm⁻¹ to 650 cm⁻¹.

### Differential Scanning Calorimetry (DSC):

Differential Scanning Calorimetry (DSC) measurements were performed with a Differential Scanning Calorimeter (DSC Q200 V23.10 Build 79, Universal V4.4A TA Instruments) equilibrated at 40°C and Ramp at a scan rate of 10°C per minute to 250°C.

### HPLC Method for measuring Chemical Purity:

The chemical purity was measured by HPLC using Shimadzu LC-2010 CHT system with UV detector or its equivalent under the following conditions: Column = Unison UK-C18, 250 mm x 4.6 mm, 3µm or Equivalent; Detector wavelength = 270 nm; Flow Rate = 0.8 ml/minute; Injection volume = 10µL; Oven temperature = 45°C; Run time = 70 minutes; Diluent = Methanol; Elution = Gradient; and Sample Concentration: 0.5 mg/ml.
Preparation of Buffer: 1000 mL of water was taken into a suitable container, followed by the addition of 1 mL of trifluoroacetic acid and then mixing the solution.
Mobile Phase-A: Buffer and Methanol (90:10 v/v).
Mobile Phase-B: Buffer and Methanol (10:90 v/v).

The following examples are given for the purpose of illustrating the present invention and should not be considered as a limitation of the invention.

### EXAMPLES

### Example 1 (reference)

### Preparation of 4-acetylamino-5-chloro-2,3-dihydrobenzofuran-7-carboxylic acid sodium salt

### Step-1: Preparation of Diethyl azodicarboxylate

Diethyl hydrazodicarboxylate (63 g) and 70-72% nitric acid solution (39.5 ml) were taken into a reaction flask at 25-30°C, and the mixture was cooled to 0-5°C. To the resulting mass, fuming nitric acid (70 ml) was added drop-wise slowly at 0-5°C and the resulting mass was stirred for 2-3 hours at the same temperature. The resulting mass was poured into a mixture of ice (150 g), water (150 ml) and dichloromethane (98 ml), followed by stirring the mass for 10 minutes at 10-15°C. The layers were separated and the aqueous layer was extracted twice with dichloromethane (98 ml x 2). The combined organic layer was washed subsequently with water (98 ml), saturated sodium bicarbonate solution (150 ml) and water (98 ml). The resulting organic layer containing diethyl azodicarboxylate is used directly in the next step.

### Step-2: Preparation of methyl 4-acetylamino-2,3-dihydrobenzo[b]furan-7-carboxylate

Methyl 4-acetylamino-2-hydroxy-3-(2-hydroxyethyl)benzoate (50 g) was taken into a reaction flask, followed by subsequent addition of tetrahydrofuran (650 ml) and triphenylphosphine (65 g) at room temperature (25-30°C). The resulting solution was stirred for 15 minutes at room temperature and then cooled the mass to 10-15°C. A solution of diethyl azodicarboxylate (39 g) in dichloromethane (260 ml), obtained in the above step-1, was slowly added to the above reaction mass at a temperature below 30°C. The resulting solution was stirred for 1 hour at room temperature. The resulting mass was filtered and washed the bed with dichloromethane (20 ml). The filtrate was distilled off to remove the solvent completely under vacuum to produce 165 g of crude methyl 4-acetylamino-2,3-dihydrobenzo[b]furan-7-carboxylate.

### Step-3: Preparation of methyl 4-acetylamino-5-chloro-2,3-dihydrobenzo[b]furan-7-carboxylate

Crude methyl 4-acetylamino-2,3-dihydrobenzo[b]furan-7-carboxylate (165 g, obtained in the above step-2) and dimethylformamide (425 ml) were taken into a reaction flask at 25-30°C and the contents were stirred for 10 minutes at the same temperature. N-Chlorosuccinimide (31.6 g) was added to the resulting mass at 25-30°C, followed by stirring the mass for 10 minutes at the same temperature. The resulting mass was heated to 70-75°C and then stirred for 2 hours at the same temperature. The reaction mass was cooled to 25-30°C, water (2125 ml) was added to the mass and then stirred for 3 hours at the same temperature. The solid obtained was filtered and washed with water (100 ml) to produce 125 g of crude methyl 4-acetylamino-5-chloro-2,3-dihydrobenzo[b]furan-7-carboxylate.

### Step-4: Preparation of 4-acetylamino-5-chloro-2,3-dihydrobenzofuran-7-carboxylic acid sodium salt

Crude methyl 4-acetylamino-5-chloro-2,3-dihydrobenzo[b]furan-7-carboxylate (125 g), obtained in the above step-3, and methanol (531.6 ml) were taken into a reaction flask at 25-30°C and the contents were stirred for 10 minutes at the same temperature. To the resulting mass, a mixture of sodium hydroxide (15.8 g) and water (98 ml) was added at 25-30°C, followed by stirring the mixture for 10 minutes at the same temperature. The resulting mass was heated to 50-55°C and then stirred for 2 hours at the same temperature. The resulting solution was cooled to 0-5°C and then stirred for 1 hour at the same temperature. The separated solid was filtered and washed with chilled methanol (50 ml) to produce 30 g of 4-acetylamino-5-chloro-2,3-dihydrobenzofuran-7-carboxylic acid sodium salt (Purity by HPLC: 98.2%).

### Example 2 (reference)

### Preparation of methyl 4-amino-5-chloro-2,3-dihydrobenzofuran-7-carboxylate

Methanol (700 ml) was taken into a reaction flask and cooled to below 10°C, followed by slow addition of sulphuric acid (215 g) at below 20°C. To the resulting solution, 4-acetylamino-5-chloro-2,3-dihydrobenzofuran-7-carboxylic acid sodium salt (140 g) was added and then stirred for 10 minutes at 25-30°C. The resulting mass was heated to reflux temperature and then stirred for 8 hours at reflux. The reaction mass was cooled to room temperature, followed by addition of water (3500 ml) at the same temperature. The resulting mass was cooled to 0-5°C and then maintained for 2 hours at the same temperature. The separated solid was filtered, washed with cold water (70 ml) to produce 80 g of methyl 4-amino-5-chloro-2,3-dihydrobenzofuran-7-carboxylate (Purity by HPLC: 98.6%).

### Example 3 (reference)

### Preparation of ethyl 4-[[(4-amino-5-chloro-2,3-dihydro-7-benzofuranyl)carbonyl] amino]-1-piperidine-carboxylate

A solution of sodium hydroxide (1.9 g) in water (10 ml) was added to methyl 4-amino-5-chloro-2,3-dihydrobenzofuran-7-carboxylate (10 g) in methanol (50 ml) at 25-30°C. The resulting mixture was heated to reflux temperature and maintained for 2 hours at the same temperature. After completion of reaction, the solvent was distilled off under vacuum to produce 4-amino-5-chloro-2,3-dihydro-7-benzofuran-carboxylic acid as a solid. Chloroform (250 ml) was added to the resulting solid and then stirred for 15 minutes at 25-30°C. The resulting mass was cooled to 0-5°C, followed by the addition of triethylamine (10 g) and ethyl chloroformate (10 g), and then stirring for 2-3 hours at the same temperature. A solution of ethyl 4-amino-1-piperidine-carboxylate (12.4 g) in chloroform (128 ml) was added to the reaction mass and the resulting mixture was maintained at 25-30°C for overnight. After completion of reaction, the salts were filtered and the filtrate was washed subsequently with 5% NaOH solution (130 ml) and water (130 ml). The solvent was distilled off under reduced pressure to obtain a residue. Diisopropyl ether (40 ml) was added to the residue and then stirred for 1 hour at 25-30°C. The product obtained was filtered, washed with di-isopropyl ether (15 ml) and then dried the material at 55-60°C for 3 hours to produce 12 g of ethyl 4-[[(4-amino-5-chloro-2,3-dihydro-7-benzofuranyl)carbonyl]amino]-1-piperidinecarboxylate (Purity by HPLC: 99.5%; Yield: 74.2%).

### Example 4

### Preparation of 4-amino-5-chloro-2,3-dihydro-N-(4-piperidinyl)-7-benzofuran carboxamide

Ethyl 4-[[(4-amino-5-chloro-2,3-dihydro-7-benzofuranyl)carbonyl]amino]-1-piperidine carboxylate (40 g) was added to a mixture of sulphuric acid (80 ml) and water (24 ml) at 10-20°C. The resulting mass was heated to 90-95°C and then stirred for 5-6 hours at the same temperature. After completion of reaction, the resulting mass was poured into a solution of ice (270 g) and water (540 ml) at 10°C. The resulting mass was stirred for 1 hour at room temperature. The resulted solid was filtered and then washed with water (30 ml). The wet product was added to water (630 ml), followed by adjusting the pH to 10-10.5 with 50% aqueous NaOH solution at 10-15°C. The reaction mass was stirred for 1 hour at 20-25°C and the product obtained was filtered, washed with water (70 ml) and then dried the material at 55-60°C for 4 hours to produce 31 g of 4-amino-5-chloro-2,3-dihydro-N-(4-piperidinyl)-7-benzofurancarboxamide (Purity by HPLC: 99.6%; Yield: 96.8%).

### Example 5

### Preparation of Prucalopride monohydrate

4-Amino-5-chloro-2,3-dihydro-N-(4-piperidinyl)-7-benzofurancarboxamide (30 g), triethyl amine (30 ml), 1-chloro-3-methoxy-propane (13.8 g) and potassium iodide (4 g) in dimethylformamide (150 ml) were taken into a reaction flask at 25-30°C. The resulting mixture was heated to 90-95°C and then maintained for 4-5 hours at the same temperature. The reaction mass was cooled to 25-30°C, followed by the addition of 1-chloro-3-methoxy-propane (5 g) and triethylamine (10 ml) at the same temperature. The reaction mass was heated to 90-95°C and then maintained for 4-5 hours at the same temperature. After completion of reaction, the reaction mass was cooled to 25-30°C, followed by the addition of water (750 ml) and then extracting with dichloromethane (300 ml x 3 times). The layers were separated and the organic layer was washed with water (500 ml). Activated carbon powder (3 g) was added to the organic layer and then stirred for 10 minutes at 25-30°C, followed by filtration through carbon bed. The resulting filtrate was concentrated under vacuum to obtain a residue. To the resulting residue, water (350 ml) was added and then stirred for 30 minutes at 20-25°C. The solid separated was filtered and washed with water (50 ml x 2 times) to produce 31 g of Prucalopride monohydrate (Purity by HPLC: 97.0%).

### Example 6 (reference)

### Preparation of Prucalopride Succinate

Ethanol (270 ml) was added to Prucalopride monohydrate (40 g; Purity by HPLC: 97%) and the resulting mass was heated to 60-65°C. After complete dissolution, activated carbon (4.0 g) was added to the reaction mass and stirred for 10 minutes at 60-65°C. The resulting mass was filtered and washed with hot ethanol (10 ml). The filtrate was heated to 60-65°C and succinic acid (13 g) was added and stirred for 15 minutes at the same temperature. After completion of reaction, the reaction mass was cooled to 20-25°C and then stirred for 1 hour at the same temperature. The separated solid was filtered, washed with ethanol (50 ml) and then dried the material at 60-65°C for 4 hours to produce 40 g of Prucalopride Succinate (Purity by HPLC: 99.0%).

### Example 7

### Preparation of Prucalopride Succinate

Methanol (135 ml) and isopropyl alcohol (135 ml) were added to prucalopride monohydrate (40 g; Purity by HPLC: 97%) and the resulting mass was heated to 60-65°C. After complete dissolution, activated carbon (4.0 g) was added to the resulting solution and the mixture was stirred for 10 minutes at 60-65°C. The resulting mass was filtered and the washed the charcoal bed with a mixture of methanol and isopropyl alcohol (10 ml, 1:1). The filtrate was heated to 60-65°C, followed by the addition of succinic acid (13 g) and then stirring the mass for 15 minutes at the same temperature. The reaction mass was cooled to 20-25°C and then stirred for 1 hour at the same temperature. The separated solid was filtered, washed with a mixture of methanol and isopropyl alcohol (50 ml, 1:1), and then dried the material at 60-65°C for 4 hours to produce 42 g of Prucalopride Succinate (Purity by HPLC: 99.0%).

### Example 8

### Preparation of Pure Prucalopride Succinate

Isopropyl alcohol (500 ml) was added to crude Prucalopride Succinate (50 g, Purity by HPLC: 99.0%) at 25-30°C and the contents were heated to reflux temperature, followed by the addition of water (40 ml) to the resulting hot solution to form a clear solution. Activated carbon powder (5 g) was added to the resulting hot solution at reflux temperature and the mixture was stirred for 10 minutes at reflux temperature and then filtered through charcoal bed. The resulting filtrate was cooled to 20-25°C, followed by stirring the mass for 1 hour at the same temperature. The separated solid was filtered, washed the solid with isopropyl alcohol (50 ml) and then dried the material for 4 hours at 55-60°C to produce 40 g of pure Prucalopride Succinate as a white to off-white crystalline powder (Purity by HPLC: 99.9%).

### Example 9

### Preparation of Pure Prucalopride Succinate

Isopropyl alcohol (500 ml) and methanol (350 ml) were added to crude Prucalopride Succinate (50 g, Purity by HPLC: 99.0%) at 25-30°C and the contents were heated to reflux temperature, followed by addition of water (40 ml to the resulting hot solution to form a clear solution. Activated carbon powder (5 g) was added to the resulting solution at reflux temperature and the mixture was stirred for 10 minutes at the same temperature. The resulting mixture was filtered through charcoal bed. The resulting filtrate was cooled to 20-25°C, followed by stirring the reaction mass for 1 hour at the same temperature. The separated solid was filtered, washed the solid with isopropyl alcohol (50 ml) and then dried the material for 4 hours at 55-60°C to produce 40 g of pure Prucalopride Succinate as a white to off-white crystalline powder (Purity by HPLC: 99.75%).

### Example 10

### Preparation of Pure Prucalopride Succinate

Isopropyl alcohol (500 ml) was added to crude Prucalopride Succinate (50 g, Purity by HPLC: 99.3%) at 25-30°C and the contents were heated to reflux temperature, followed by stirring the resulting mass at reflux for 30 minutes. The resulting mass was cooled to 25-30°C and then stirred for 1 hour at the same temperature. The resulting material was filtered, washed the solid with isopropyl alcohol (50 ml) and then dried the material for 4 hours at 55-60°C to produce 45 g of Prucalopride Succinate (Purity by HPLC: 99.7%).

### Example 11

### Preparation of Pure Prucalopride Succinate

Acetone (75 ml) was added to crude Prucalopride Succinate (5 g, Purity by HPLC: 99.0%) at 25-30°C, the contents were heated to reflux temperature and then stirred for 15 minutes at the same temperature. Water (13 ml) was added to the resulting hot solution to form a clear solution. Activated carbon powder (0.5 g) was added to the resulting hot solution at reflux temperature and then stirred for 10 minutes at the same temperature. The reaction mixture was filtered through charcoal bed. The resulting filtrate was cooled to 5-10°C, followed by stirring the reaction mass for 30 minutes at the same temperature. The separated solid was filtered, washed the solid with chilled acetone (10 ml) and then dried the material for 3 hours at 50-55°C to produce 4 g of pure Prucalopride Succinate (Purity by HPLC: 99.65%).

## Claims

1. A process for purification of Prucalopride succinate, comprising:
a) providing a solution of Prucalopride succinate in a solvent medium comprising an organic solvent and water at a temperature of above 40°C, wherein the solvent medium used is selected from the group consisting of a solvent medium comprising isopropyl alcohol and water; a solvent medium comprising isopropyl alcohol, methanol and water; and a solvent medium comprising acetone and water;
b) subjecting the solution obtained in step-(a) to carbon treatment to obtain a clear filtrate;
c) cooling the filtrate obtained in step-(b) at a temperature below 35°C to cause crystallization; and
d) recovering the highly pure Prucalopride Succinate obtained in step-(c).

2. The process of claim 1, wherein the Prucalopride succinate obtained is further **characterized by** an X-ray powder diffraction pattern having peaks expressed as 2-theta angle positions at 7.75, 9.18, 9.97, 10.12, 13.65, 13.77, 15.33, 15.52, 15.80, 18.39, 19.71, 19.96, 20.23, 20.41, 20.85, 22.20, 22.40, 23.36, 24.12, 24.65, 25.17, 27.38, 27.67, 28.37, 28.81, 29.92, 31.13, 41.58, 42.79, 43.20 and 45.24 ± 0.2 degrees using copper-Kα radiation; an infra red (FT-IR) spectrum having main bands at 3446, 3397, 3327, 3217, 2979, 2914, 2806, 1650, 1635, 1609, 1577, 1539, 1488, 1448, 1429, 1401, 1348, 1320, 1252, 1224, 1187, 1164, 1148, 1114, 1069, 1050, 998, 970, 938, 903, 774 and 728 cm⁻¹ ± 2 cm⁻¹.

3. The process of claim 1, wherein the amount of solvent medium employed in step-(a) is 5 volumes to 15 volumes with respect to the quantity of crude Prucalopride succinate used; wherein the crystallization in step-(c) is accomplished by cooling the solution at a temperature of 0°C to 35°C; and wherein the recovering in step-(d) is carried out by filtration, filtration under vacuum, decantation, centrifugation or a combination thereof.

4. The process of claim 3, wherein the solvent medium employed in step-(a) is a solvent medium comprising isopropyl alcohol and water, and wherein the amount of isopropyl alcohol employed is 5 to 15 volumes with respect to the volume of water used.

5. A process for the preparation of Prucalopride succinate, which comprises:
a) dissolving Prucalopride monohydrate in a solvent medium comprising methanol and isopropyl alcohol at a temperature of 55°C to 65°C to form a clear solution;
b) subjecting the solution obtained in step-(a) to carbon treatment to obtain a clear filtrate at a temperature of 55°C to 65°C to form a clear solution;
c) combining the filtrate obtained in step-(b) with succinic acid; and
d) recovering the Prucalopride Succinate obtained in step-(c).

6. The process of claim 5, wherein the solution in step-(a) is prepared by dissolving Prucalopride monohydrate in the solvent medium comprising methanol and isopropyl alcohol at a temperature of 60°C to 65°C; and wherein the recovering in step-(d) is carried out by filtration, filtration under vacuum, decantation, centrifugation or a combination thereof.

7. The process as claimed in claim 5, further comprising preparing Prucalopride of formula 1 used in step (a) of claim 5: 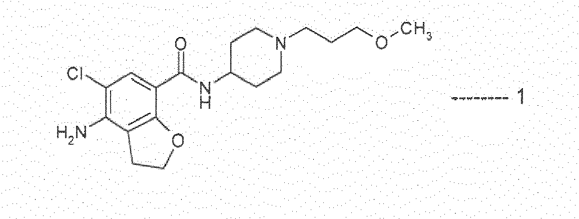 or a pharmaceutically acceptable salt thereof, by a process which comprises:
a) deprotecting the alkyl 4-[[(4-amino-5-chloro-2,3-dihydro-7-benzofuranyl)-carbonyl]-amino]-1-piperidinecarboxylate of formula 3: 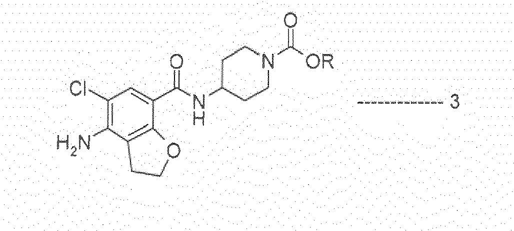
or a salt thereof, wherein R is an alkyl group having 1 to 5 carbon atoms,
with an acid in a suitable solvent to produce 4-amino-5-chloro-2,3-dihydro-N-(4-piperidinyl)-7-benzofurancarboxamide of formula 2: 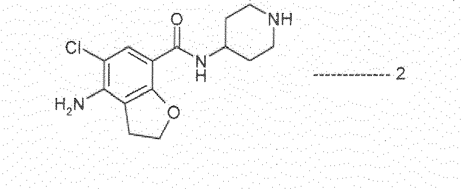
or a salt thereof; and
b) reacting the compound of formula 2 with 1-chloro-3-methoxy-propane in presence of a base, optionally in presence of a suitable catalyst, to produce Prucalopride of formula 1 or a pharmaceutically acceptable salt thereof.

8. The process of claim 7, wherein the group R in the compound of formula 3 is selected from methyl, ethyl, n-propyl, isopropyl, n-butyl, iso-butyl, tert-butyl and n-pentyl; wherein the acid used for deprotection in step-(a) is selected from the group consisting of hydrochloric acid, hydrobromic acid, sulfuric acid, nitric acid, phosphoric acid, acetic acid, formic acid, trifluoroacetic acid and trifluoroacetic anhydride; wherein the solvent used for deprotection in step-(a) is selected from the group consisting of water, methanol, ethanol, isopropyl alcohol, tert-butyl alcohol, N,N-dimethylformamide, N,N-dimethylacetamide, dichloromethane, dichloroethane, dioxane, and mixtures thereof; wherein the reaction in step-(b) is carried out in the presence of a solvent or a mixture of solvents; wherein the base used in step-(b) is an organic base; and wherein the reaction in step-(b) is carried out in the presence of a suitable catalyst.

9. The process of claim 8, wherein the group R in the compound of formula 3 is ethyl; wherein the acid used for deprotection in step-(a) is sulfuric acid; wherein the solvent used for deprotection in step-(a) is water; wherein the solvent used in step-(b) is selected from the group consisting of toluene, xylene, N,N-dimethylformamide, N,N-dimethylacetamide, dichloromethane, dimethylsulfoxide, N-methylpyrrolidone and mixtures thereof; wherein the organic base used in step-(b) is selected from the group consisting of methylamine, trimethylamine, tributylamine, triethylamine, diisopropylethylamine, N-methylmorpholine, and 1-alkylimidazole; and wherein the catalyst used in step-(b) is sodium iodide or potassium iodide.

10. The process as claimed in claim 7, comprising preparing alkyl 4-[[(4-amino-5-chloro-2,3-dihydro-7-benzofuranyl)carbonyl]-amino)-1-piperidinecarboxylate of formula 3: 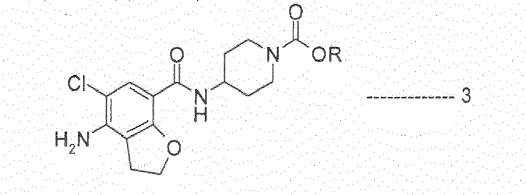 or a salt thereof, wherein R is an alkyl group having 1 to 5 carbon atoms, by a process which comprises:
a) reacting methyl 4-acetylamino-5-chloro-2,3-dihydrobenzo[b]furan-7-carboxylate of formula 7: 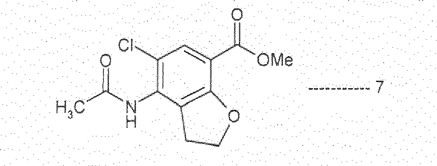 or a salt thereof, with an alkali metal base to produce 4-acetylamino-5-chloro-2,3-dihydrobenzofuran-7-carboxylic acid alkali metal salt of formula 6: 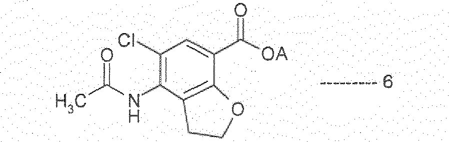 wherein the radical 'A' is an alkali metal;
b) reacting the compound of formula 6 with an acid in presence of methanol as a solvent to produce methyl 4-amino-5-chloro-2,3-dihydrobenzofuran-7-carboxylate of formula 5: 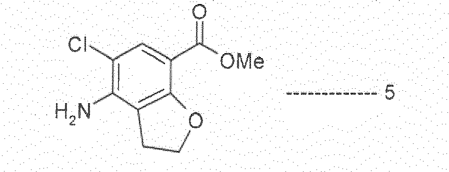 or an acid addition salt thereof;
c) hydrolysis of the compound of formula 5 or an acid addition salt thereof with a suitable inorganic base to produce 4-amino-5-chloro-2,3-dihydro-7-benzofuran-carboxylic acid of formula 4: 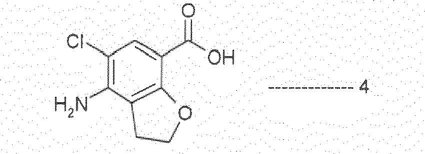 or a salt thereof; and
d) reacting the compound of formula 4 or a salt thereof with an alkyl 4-amino-1-piperidine-carboxylate of formula 10: 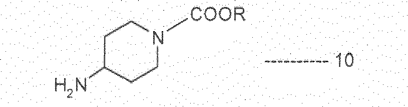 wherein R is as defined above for formula 3, in presence of ethyl chloroformate and an organic base to produce alkyl 4-[[(4-amino-5-chloro-2,3-dihydro-7-benzofuranyl)carbonyl]-amino]-1-piperidinecarboxylate of formula 3 or a salt thereof.

11. The process of claim 10, wherein the group R in the compounds of formulae 3 and 10 is selected from methyl, ethyl, n-propyl, isopropyl, n-butyl, iso-butyl, tert-butyl, and n-pentyl; and wherein the radical 'A' in the compound of formula 6 is sodium or potassium,

12. The process of claim 10, wherein the alkali metal base used in step-(a) is selected from the group consisting of sodium hydroxide, potassium hydroxide, lithium hydroxide, sodium carbonate, potassium carbonate, sodium bicarbonate, potassium bicarbonate, lithium carbonate; wherein the solvent used in step-(a) is selected from the group consisting of water, methanol, ethanol, isopropyl alcohol, n-butyl alcohol, tert-butyl alcohol, and mixtures thereof; wherein the acid used for deprotection in step-(b) is an organic or an inorganic acid selected from the group consisting of hydrochloric acid, hydrobromic acid, sulfuric acid, nitric acid, phosphoric acid, acetic acid, formic acid, trifluoroacetic acid and trifluoroacetic anhydride; wherein the reaction in step-(c) is carried out in the presence of a solvent selected from the group consisting of water, methanol, ethanol, isopropyl alcohol, n-butyl alcohol, tert-butyl alcohol, and mixtures thereof; wherein the inorganic base used for hydrolysis in step-(c) is sodium hydroxide or potassium hydroxide; wherein the reaction in step-(d) is carried out in the presence of a solvent or a mixture of solvents; and wherein the organic base used in step-(d) is selected from the group consisting of methylamine, trimethylamine, tributylamine, triethylamine, diisopropylethylamine, N-methylmorpholine and 1-alkylimidazole.

## Patentansprüche

1. Verfahren zur Reinigung von Prucalopridsuccinat, umfassend:
a) Bereitstellen einer Lösung von Prucalopridsuccinat in einem Lösungsmittelmedium, umfassend ein organisches Lösungsmittel und Wasser bei einer Temperatur von über 40 °C, wobei das verwendete Lösungsmittelmedium aus der Gruppe ausgewählt ist, die aus einem Lösungsmittelmedium, umfassend Isopropylalkohol und Wasser; einem Lösungsmittelmedium, umfassend Isopropylalkohol, Methanol und Wasser, und einem Lösungsmittelmedium, umfassend Aceton und Wasser; besteht;
b) Unterziehen der in Schritt-(a) erhaltenen Lösung einer Kohlenstoffbehandlung unter Erhalt eines klaren Filtrats;
c) Abkühlen des in Schritt-(b) erhaltenen Filtrats bei einer Temperatur unter 35 °C, um Kristallisation zu bewirken; und
d) Gewinnen des in Schritt-(c) erhaltenen hochreinen Prucalopridsuccinats.

2. Verfahren nach Anspruch 1, wobei das erhaltene Prucalopridsuccinat ferner durch ein Röntgenpulverbeugungsdiagramm mit Peaks, ausgedrückt als 2-theta-Winkelpositionen bei 7,75, 9,18, 9,97, 10,12, 13,65, 13,77, 15,33, 15,52, 15,80, 18,39, 19,71, 19,96, 20,23, 20,41, 20,85, 22,20, 22,40, 23,36, 24,12, 24,65, 25,17, 27,38, 27,67, 28,37, 28,81, 29,92, 31,13, 41,58, 42,79, 43,20 und 45,24 ± 0,2 Grad, unter Verwendung von Kupfer-Kα-Strahlung; ein Infrarot (FT-IR)-Spektrum mit Hauptbanden bei 3.446, 3.397, 3.327, 3.217, 2.979, 2.914, 2.806, 1.650, 1.635, 1.609, 1.577, 1.539, 1.488, 1.448, 1.429, 1.401, 1.348, 1.320, 1.252, 1.224, 1.187, 1.164, 1.148, 1.114, 1.069, 1.050, 998, 970, 938, 903, 774 und 728 cm⁻¹ ± 2 cm⁻¹ gekennzeichnet ist.

3. Verfahren nach Anspruch 1, wobei die in Schritt-(a) eingesetzte Menge an Lösungsmittelmedium 5 Volumen bis 15 Volumen in Bezug auf die Menge an verwendetem rohem Prucalopridsuccinat beträgt; wobei die Kristallisation in Schritt-(c) durch Abkühlen der Lösung bei einer Temperatur von 0 °C bis 35 °C bewerkstelligt wird; und wobei das Gewinnen in Schritt-(d) durch Filtration, Filtration unter Vakuum, Dekantierung, Zentrifugation oder eine Kombination davon erfolgt.

4. Verfahren nach Anspruch 3, wobei das in Schritt-(a) eingesetzte Lösungsmittelmedium ein Lösungsmittelmedium ist, das Isopropylalkohol und Wasser umfasst, und wobei die eingesetzte Menge an Isopropylalkohol 5 bis 15 Volumen in Bezug auf das verwendete Volumen von Wasser beträgt.

5. Verfahren zur Herstellung von Prucalopridsuccinat, welches:
a) das Auflösen von Prucalopridmonohydrat in einem Lösungsmittelmedium, das Methanol und Isopropylalkohol umfasst, bei einer Temperatur von 55 °C bis 65 °C unter Bildung einer klaren Lösung;
b) das Unterziehen der in Schritt-(a) erhaltenen Lösung einer Kohlenstoffbehandlung unter Erhalt eines klaren Filtrats bei einer Temperatur von 55 °C bis 65 °C unter Bildung einer klaren Lösung;
c) das Vereinigen des in Schritt-(b) erhaltenen Filtrats mit Bernsteinsäure; und
d) das Gewinnen des in Schritt-(c) erhaltenen Prucalopridsuccinats umfasst.

6. Verfahren nach Anspruch 5, wobei die Lösung in Schritt-(a) durch Auflösung von Prucalopridmonohydrat in dem Lösungsmittelmedium, das Methanol und Isopropylalkohol umfasst, bei einer Temperatur von 60 °C bis 65 °C hergestellt wird; und wobei das Gewinnen in Schritt-(d) durch Filtration, Filtration unter Vakuum, Dekantierung, Zentrifugation oder eine Kombination davon erfolgt.

7. Verfahren nach Anspruch 5, ferner umfassend das Herstellen von Prucaloprid der Formel 1, das in Schritt (a) von Anspruch 5 verwendet wird: 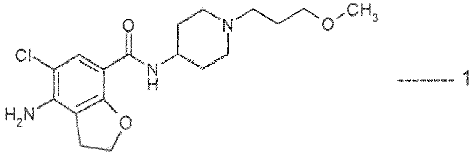 oder eines pharmazeutisch akzeptablen Salzes davon, mittels eines Verfahrens, welches:
a) das Entschützen des Alkyl-4-[[(4-amino-5-chlor-2,3-dihydro-7-benzofuranyl)-carbonyl]-amino]-1-piperidincarboxylats der Formel 3: 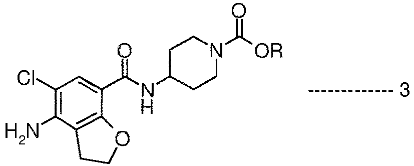 oder eines Salzes davon, wobei R eine Alkylgruppe mit 1 bis 5 Kohlenstoffatomen ist, mit einer Säure in einem geeigneten Lösungsmittel unter Herstellung von 4-Amino-5-chlor-2,3-dihydro-N-(4-piperidinyl)-7-benzofurancarboxamid der Formel 2: 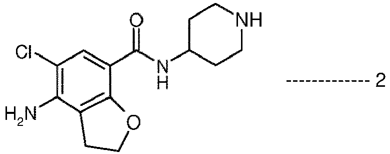 oder eines Salzes davon; und
b) das Umsetzen der Verbindung der Formel 2 mit 1-Chlor-3-methoxy-propan in Gegenwart einer Base, gegebenenfalls in Gegenwart eines geeigneten Katalysators, unter Herstellung von Prucaloprid der Formel 1 oder eines pharmazeutisch akzeptablen Salzes davon
umfasst.

8. Verfahren nach Anspruch 7, wobei die Gruppe R in der Verbindung von Formel 3 aus Methyl, Ethyl, n-Propyl, Isopropyl, n-Butyl, iso-Butyl, tert-Butyl und n-Pentyl ausgewählt ist; wobei die zur Entschützung in Schritt-(a) verwendete Säure aus der Gruppe ausgewählt ist, die aus Chlorwasserstoffsäure, Bromwasserstoffsäure, Schwefelsäure, Salpetersäure, Phosphorsäure, Essigsäure, Ameisensäure, Trifluoressigsäure und Trifluoressigsäureanhydrid besteht; wobei das zum Entschützen in Schritt-(a) verwendete Lösungsmittel aus der Gruppe ausgewählt ist, die aus Wasser, Methanol, Ethanol, Isopropylalkohol, tert-Butylalkohol, N,N-Dimethylformamid, N,N-Dimethylacetamid, Dichlormethan, Dichlorethan, Dioxan und Gemischen davon besteht; wobei die Umsetzung in Schritt-(b) in der Gegenwart eines Lösungsmittels oder eines Gemisches aus Lösungsmitteln durchgeführt wird; wobei die in Schritt-(b) verwendete Base eine organische Base ist und wobei die Umsetzung in Schritt-(b) in der Gegenwart eines geeigneten Katalysators durchgeführt wird.

9. Verfahren nach Anspruch 8, wobei die Gruppe R in der Verbindung der Formel 3 Ethyl ist; wobei die zum Entschützen in Schritt-(a) verwendete Säure Schwefelsäure ist; wobei das zum Entschützen in Schritt-(a) verwendete Lösungsmittel Wasser ist; wobei das in Schritt-(b) verwendete Lösungsmittel aus der Gruppe ausgewählt ist, die aus Toluol, Xylol, N,N-Dimethylformamid, N,N-Dimethylacetamid, Dichlormethan, Dimethylsulfoxid, N-Methylpyrrolidon und Gemischen davon besteht; wobei die in Schritt-(b) verwendete organische Base aus der Gruppe ausgewählt ist, die aus Methylamin, Trimethylamin, Tributylamin, Triethylamin, Diisopropylethylamin, N-Methylmorpholin und 1-Alkylimidazol besteht, und wobei der in Schritt-(b) verwendete Katalysator Natriumiodid oder Kaliumiodid ist.

10. Verfahren nach Anspruch 7, umfassend das Herstellen von Alkyl-4-[[(4-amino-5-chlor-2,3-dihydro-7-benzofuranyl)carbonyl]-amino]-1-piperidincarboxylat der Formel 3: 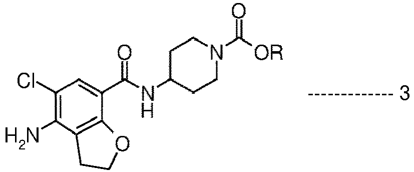 oder eines Salzes davon, wobei R eine Alkylgruppe mit 1 bis 5 Kohlenstoffatomen ist, mittels eines Verfahrens, welches:
a) das Umsetzen von Methyl-4-acetylamino-5-chlor-2,3-dihydrobenzo[b]furan-7-carboxylat der Formel 7: 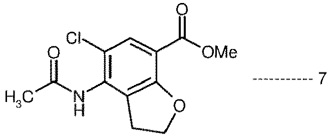 oder eines Salzes davon mit einer Alkalimetallbase zur Herstellung des 4-Acetylamino-5-chlor-2,3-dihydrobenzofuran-7-carbonsäure-Alkalimetallsalzes der Formel 6: 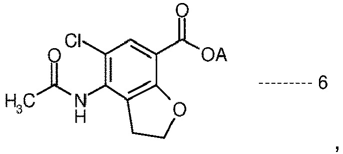 wobei der Rest "A" ein Alkalimetall ist;
b) das Umsetzen der Verbindung der Formel 6 mit einer Säure in Gegenwart von Methanol als Lösungsmittel zur Herstellung von Methyl-4-amino-5-chlor-2,3-dihydrobenzofuran-7-carboxylat der Formel 5: 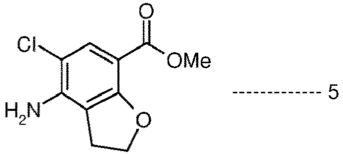 oder eines Säureadditionssalzes davon;
c) die Hydrolyse der Verbindung der Formel 5 oder eines Säureadditionssalzes davon mit einer geeigneten anorganischen Base zur Herstellung von 4-Amino-5-chlor-2,3-dihydro-7-benzofuran-carbonsäure der Formel 4: 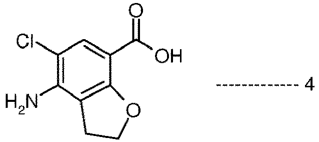 oder eines Salzes davon; und
d) das Umsetzen der Verbindung der Formel 4 oder eines Salzes davon mit einem Alky-4-amino-1-piperidincarboxylat der Formel 10: 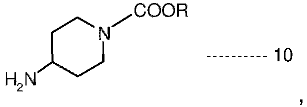 wobei R wie oben für Formel 3 definiert ist, in Gegenwart von Ethylchlorformiat und einer organischen Base zur Herstellung des Alkyl-4-[[(4-amino-5-chlor-2,3-dihydro-7-benzofuranyl)carbonyl]-amino]-1-piperidincarboxylats der Formel 3 oder eines Salzes davon
umfasst.

11. Verfahren nach Anspruch 10, wobei die Gruppe R in den Verbindungen der Formeln 3 und 10 aus Methyl, Ethyl, n-Propyl, Isopropyl, n-Butyl, iso-Butyl, tert-Butyl und n-Pentyl ausgewählt ist und wobei der Rest "A" in der Verbindung der Formel 6 Natrium oder Kalium ist.

12. Verfahren nach Anspruch 10, wobei die in Schritt-(a) verwendete Alkalimetallbase aus der Gruppe ausgewählt ist, die aus Natriumhydroxid, Kaliumhydroxid, Lithiumhydroxid, Natriumcarbonat, Kaliumcarbonat, Natriumbicarbonat, Kaliumbicarbonat, Lithiumcarbonat besteht; wobei das in Schritt-(a) verwendete Lösungsmittel aus der Gruppe ausgewählt ist, die aus Wasser, Methanol, Ethanol, Isopropylalkohol, n-Butylalkohol, tert-Butylalkohol und Gemischen davon besteht; wobei die zum Entschützen in Schritt-(b) verwendete Säure eine organische oder eine anorganische Säure ist, die aus der Gruppe ausgewählt ist, die aus Chlorwasserstoffsäure, Bromwasserstoffsäure, Schwefelsäure, Salpetersäure, Phosphorsäure, Essigsäure, Ameisensäure, Trifluoressigsäure und Trifluoressigsäureanhydrid besteht; wobei die Umsetzung in Schritt-(c) in der Gegenwart eines Lösungsmittels durchgeführt wird, das aus der Gruppe ausgewählt ist, die aus Wasser, Methanol, Ethanol, Isopropylalkohol, n-Butylalkohol, tert-Butylalkohol und Gemischen davon besteht; wobei die zur Hydrolyse in Schritt-(c) verwendete anorganische Base Natriumhydroxid oder Kaliumhydroxid ist; wobei die Umsetzung in Schritt-(d) in der Gegenwart eines Lösungsmittels oder eines Gemisches aus Lösungsmitteln durchgeführt wird und wobei die in Schritt-(d) verwendete organische Base aus der Gruppe ausgewählt ist, die aus Methylamin, Trimethylamin, Tributylamin, Triethylamin, Diisopropylethylamin, N-Methylmorpholin und 1-Alkylimidazol besteht.

## Revendications

1. Procédé de purification du succinate de Prucalopride comprenant les étapes consistant à :
a) fournir une solution de succinate de Prucalopride dans un milieu solvant comprenant un solvant organique et de l'eau à une température supérieure à 40°C, dans lequel le milieu solvant utilisé est choisi dans le groupe constitué d'un milieu solvant comprenant de l'alcool isopropylique et de l'eau, d'un milieu solvant comprenant de l'alcool isopropylique, du méthanol et de l'eau, et d'un milieu solvant comprenant de l'acétone et de l'eau ;
b) soumettre la solution obtenue à l'étape a) à un traitement au charbon pour obtenir un filtrat clair ;
c) refroidir le filtrat obtenu à l'étape (b) à une température inférieure à 35°C pour provoquer la cristallisation ; et
d) récupérer le succinate de Prucalopride très pur obtenu à l'étape c).

2. Procédé de la revendication 1, dans lequel le succinate de Prucalopride obtenu est en outre **caractérisé par** un diagramme de diffraction de rayons X sur poudre présentant des pics exprimés en positions d'angle 2-thêta à 7,75, 9,18, 9,97, 10,12, 13,65, 13,77, 15,33, 15,52, 15,80, 18,39, 19,71, 19,96, 20,23, 20,41, 20,85, 22,20, 22,40, 23,36, 24,12, 24,65, 25,17, 27,38, 27,67, 28,37, 28,81, 29,92, 31,13, 41,58, 42,79, 43,20 et 45,24 ±0.2 degrés en utilisant la radiation cuivre-Kα; un spectre infrarouge (FT-IR) ayant des bandes principales à 3446, 3397, 3327, 3217, 2979, 2914, 2806, 1650, 1635, 1609, 1577, 1539, 1488, 1448, 1429, 1401, 1348, 1320, 1252, 1224, 1187, 1164, 1148, 1114, 1069, 1050, 998, 970, 938, 903, 774 et 728cm⁻¹ ± 2cm⁻¹.

3. Procédé selon la revendication 1, dans lequel la quantité de milieu solvant utilisée à l'étape (a) est de 5 à 15 volumes par rapport à la quantité de succinate de Prucalopride brut utilisée; dans lequel la cristallisation à l'étape (c) est accomplie en refroidissant la solution à une température de 0°C à 35°C ; et dans lequel la récupération à l'étape (d) est effectuée par filtration, filtration sous vide, décantation, centrifugation ou une combinaison de ces procédés.

4. Procédé selon la revendication 3, dans lequel le solvant utilisé à l'étape (a) est un milieu solvant comprenant de l'alcool isopropylique et de l'eau, et dans lequel la quantité d'alcool isopropylique utilisée est de 5 à 15 volumes par rapport au volume d'eau utilisé.

5. Procédé de préparation du succinate de Prucalopride, qui comprend les étapes consistant à :
a) dissoudre le Prucalopride monohydraté dans un milieu solvant comprenant du méthanol et de l'alcool isopropylique à une température de 55°C à 65°C pour former une solution claire ;
b) soumettre la solution obtenue à l'étape a) à un traitement au charbon pour obtenir un filtrat clair à une température de 55°C à 65°C pour former une solution claire ;
c) combiner le filtrat obtenu à l'étape b) avec de l'acide succinique ; et
d) récupérer le succinate de Prucalopride obtenu à l'étape c).

6. Procédé selon la revendication 5, dans lequel la solution de l'étape (a) est préparée en dissolvant le Prucalopride monohydraté dans le milieu solvant comprenant le méthanol et l'alcool isopropylique à une température de 60°C à 65°C ; et dans lequel la récupération de l'étape (d) est effectuée par filtration, filtration sous vide, décantation, centrifugation ou une combinaison de ces procédés.

7. Procédé selon la revendication 5, comprenant en outre la préparation du Prucalopride de formule 1 utilisé à l'étape (a) de la revendication 5 : 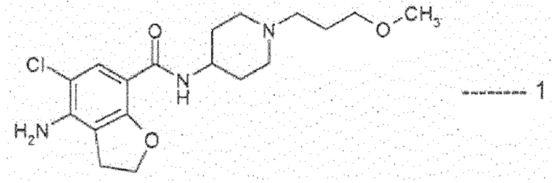 ou un de ses sels pharmaceutiquement acceptables, par un procédé qui comprend les étapes consistant à :
a) déprotéger le 4-[[(4-amino-5-chloro-2,3-dihydro-7-benzofuranyl)-carbonyl]-amino]-1-pipéridinecarboxylate d'alkyle de formule 3 : 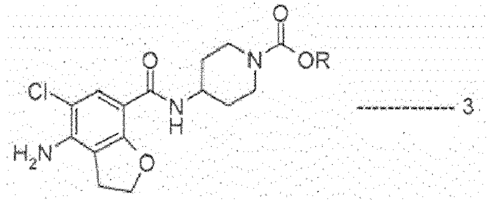
ou un de ses sels, dans lequel R représente un groupe alkyle ayant de 1 à 5 atomes de carbone,
avec un acide dans un solvant approprié pour produire le 4-amino-5-chloro-2,3-dihydro-N-(4-piperidinyl)-7-benzofurancarboxamide de formule 2 : 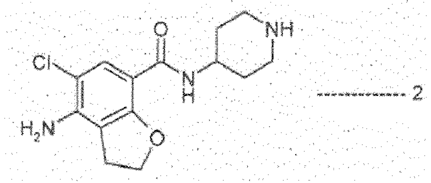
ou un de ses sels ; et
b) faire réagir le composé de formule 2 avec le 1-chloro-3-méthoxy-propane en présence d'une base, éventuellement en présence d'un catalyseur approprié, pour produire le Prucalopride de formule 1 ou un de ses sels pharmaceutiquement acceptables.

8. Procédé selon la revendication 7, dans lequel le groupe R dans le composé de formule 3 est choisi parmi le méthyle, l'éthyle, le n-propyle, l'isopropyle, le n-butyle, l'isobutyle, le tert-butyle et le n-pentyle ; dans lequel l'acide utilisé pour la déprotection à l'étape a) est choisi dans le groupe constitué de l'acide chlorhydrique, de l'acide bromhydrique, de l'acide sulfurique, de l'acide nitrique, de l'acide phosphorique, de l'acide acétique, de l'acide formique, de l'acide trifluoroacétique et de l'anhydride trifluoroacétique ; dans lequel le solvant utilisé pour la déprotection à l'étape a) est choisi dans le groupe constitué de l'eau, du méthanol, l'éthanol, l'alcool isopropylique, l'alcool tert-butylique, le N-N-diméthylformamide, le N-N diméthylacétamide, le dichlorométhane, le dichloroéthane, le dioxane et leurs mélanges ; dans lequel la réaction à l'étape b) est effectuée en présence d'un solvant ou d'un mélange de solvants ; dans lequel la base utilisée à l'étape b) est une base organique ; et dans lequel la réaction à l'étape b) est effectuée en présence d'un catalyseur approprié.

9. Procédé selon la revendication 8, dans lequel le groupe R dans le composé de formule 3 est l'éthyle ; dans lequel l'acide utilisé pour la déprotection à l'étape (a) est l'acide sulfurique ; dans lequel le solvant utilisé pour la déprotection à l'étape (a) est l'eau ; dans lequel le solvant utilisé à l'étape b) est choisi dans le groupe constitué du toluène, le xylène, le N,N-diméthylformamide, le N,N-diméthylacétamide, le dichlorométhane, le diméthylsulfoxyde, le N-méthylpyrrolidone et leurs mélanges ; dans lequel la base organique utilisée à l'étape (b) est choisie dans le groupe constitué de la méthylamine, la triméthylamine, la tributylamine, la triéthylamine, la diisopropyléthylamine, la N-méthylmorpholine et le 1-alkylimidazole ; et dans lequel le catalyseur utilisé à l'étape (b) est l'iodure de sodium ou l'iodure de potassium.

10. Procédé selon la revendication 7, comprenant la préparation du 4-[[(4-amino-5-chloro-2,3-dihydro-7-benzofuranyl)carbonyl]-amino]-1-pipéridinecarboxylate d'alkyle de formule 3 : 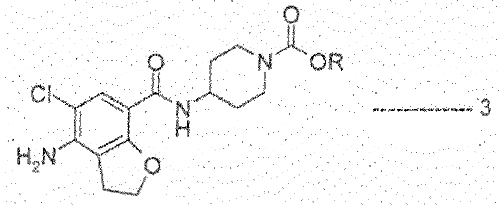 ou un de ses sels, dans lequel R représente un groupe alkyle ayant de 1 à 5 atomes de carbone, par un procédé qui comprend :
a) la réaction du 4-acétylamino-5-chloro-2,3-dihydrobenzo[b]furan-7-carboxylate de méthyle de formule 7 : 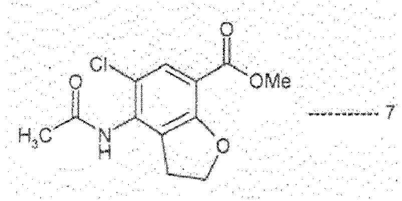
ou un de ses sels, avec une base de métal alcalin pour produire le sel de métal alcalin de l'acide 4-acétylamino-5-chloro-2,3-dihydrobenzofuran-7-carboxylique de formule 6 : 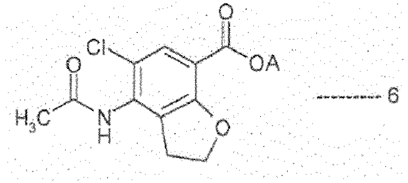
où le radical « A » représente un métal alcalin ;
b) la réaction du composé de formule 6 avec un acide en présence de méthanol comme solvant pour produire le 4-amino-5-chloro-2,3-dihydrobenzofuran-7-carboxylate de méthyle de formule 5 : 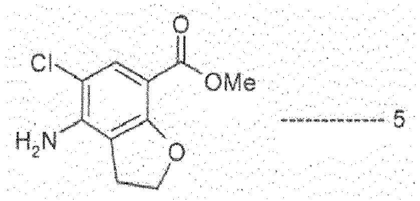 ou un de ses sels d'addition d'acide ;
c) l'hydrolyse du composé de formule 5 ou de son sel d'addition acide avec une base inorganique appropriée pour produire l'acide 4-amino-5-chloro-2,3-dihydro-7-benzofuran-carboxylique de formule 4 : 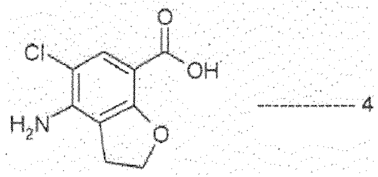 ou un de ses sels ; et
d) la réaction du composé de formule 4 ou d'un de ses sels avec un carboxylate d'alkyle 4-amino-1-pipéridine de formule 10 : 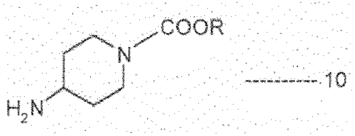 dans lequel R représente tel que défini ci-dessus pour la formule 3, en présence de chloroformate d'éthyle et d'une base organique pour produire le 4-[[(4-amino-5-chloro-2,3-dihydro-7-benzofuranyl)carbonyl]-amino]-1-pipéridinecarboxylate d'alkyle de formule 3 ou un de ses sels.

11. Procédé selon la revendication 10, dans lequel le groupe R dans les composés des formules 3 et 10 est choisi parmi le méthyle, l'éthyle, le n-propyle, l'isopropyle, le n-butyle, l'iso-butyle, le tert-butyle et le n-pentyle ; et dans lequel le radical « A » dans le composé de la formule 6 est le sodium ou le potassium.

12. Procédé selon la revendication 10, dans lequel la base alcaline utilisée à l'étape (a) est choisie dans le groupe constitué de l'hydroxyde de sodium, l'hydroxyde de potassium, l'hydroxyde de lithium, le carbonate de sodium, le carbonate de potassium, le bicarbonate de sodium, le bicarbonate de potassium, le carbonate de lithium, dans lequel le solvant utilisé à l'étape (a) est choisi dans le groupe constitué de l'eau, le méthanol, l'éthanol, l'alcool isopropylique, l'alcool n-butylique, l'alcool tert-butylique et les mélanges de ceux-ci ; dans lequel l'acide utilisé pour la déprotection à l'étape b) est un acide organique ou inorganique choisi dans le groupe constitué de l'acide chlorhydrique, de l'acide bromhydrique, de l'acide sulfurique, de l'acide nitrique, de l'acide phosphorique, de l'acide acétique, de l'acide formique, de l'acide trifluoroacétique et de l'anhydride trifluoroacétique ; dans lequel la réaction à l'étape c) est effectuée en présence d'un solvant choisi dans le groupe constitué de l'eau, le méthanol, l'éthanol, l'alcool isopropylique, l'alcool n-butylique, l'alcool tert-butylique et leurs mélanges ; dans lequel la base inorganique utilisée pour l'hydrolyse à l'étape c) est l'hydroxyde de sodium ou l'hydroxyde de potassium ; dans lequel la réaction à l'étape d) est effectuée en présence d'un solvant ou d'un mélange de solvants ; et dans lequel la base organique utilisée à l'étape d) est choisie dans le groupe constitué de la méthylamine, de la triméthylamine, de la tributylamine, de la triéthylamine, de la diisopropyléthylamine, de la N-méthylmorpholine et du 1-alkylimidazole.
